# EUROPEAN PATENT APPLICATION

(11) **EP 4 431 061 A2**
(43) Date of publication of application: **18.09.2024**
(21) Application number: 24171778.4
(22) Date of filing: 21.05.2019
(51) Int. Cl.: A61F 2/24

(54) **IMPLANTABLE ANNULOPLASTY STRUCTURES TO FIT MULTIPLE ANNULUS SIZES**

(30) Priority: 24.05.2018 US 201862676145 P; 21.08.2018 US 201862720392 P
(62) Divisional of application: 19728139.7
(71) Applicant: Edwards Lifesciences Innovation (Israel) Ltd., 60376 Or Yehuda (IL)
(72) Inventor: Peleg, Carmel, IRVINE, CA, 92614 (US); Peer, Amit, IRVINE, CA, 92614 (US); Kutzik, Meir, IRVINE, CA, 92614 (US); Fogel, Alon, IRVINE, CA, 92614 (US); Azouri, Ben, IRVINE, CA, 92614 (US); Brauon, Haim, IRVINE, CA, 92614 (US); Chappel-Ram, Shlomit, IRVINE, CA, 92614 (US); Reich, Tal, IRVINE, CA, 92614 (US)
(74) Representative: Eisenführ Speiser

(57) **Abstract**

An annuloplasty structure (122) includes a body portion (123) and a contracting member (30). The body portion can include flexible material and be shaped to define one or more controllably-expandable sections (40). Each of the controllably-expandable sections has a respective first length (L1). Each of the controllably-expandable sections can be expanded or reduced to assume a respective second length (L2) that is different from the respective first length. The contracting member is coupled to the body portion, and is configured to contract the body portion independently of the mechanism that expands the controllably-expandable sections. Other embodiments are also described.

## Description

### CROSS-REFERENCES TO RELATED APPLICATIONS

The present application claims priority from US Provisional Patent Application 62/676,145 to Peleg et al., filed on May 24, 2018; and US Provisional Patent Application 62/720,392 to Peleg et al., filed on August 21, 2018. Each of the above applications is incorporated herein by reference.

### FIELD OF THE INVENTION

Some applications relate in general to valve repair, for example, repair of an atrioventricular valve of a patient. Some applications relate to minimally invasive and/or transluminal valve repair.

### BACKGROUND

Ischemic heart disease can cause mitral regurgitation by the combination of ischemic dysfunction of the papillary muscles, and the dilatation of the left ventricle that is present in ischemic heart disease, with the subsequent displacement of the papillary muscles and the dilatation of the mitral valve annulus.

Dilation of the annulus of the mitral valve prevents the valve leaflets from fully coapting when the valve is closed. Mitral regurgitation of blood from the left ventricle into the left atrium results in increased total stroke volume and decreased cardiac output, and ultimate weakening of the left ventricle secondary to a volume overload and a pressure overload of the left atrium. Various problems can also occur with regurgitation at the tricuspid valve and other valves.

### SUMMARY

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. The description herein relates to systems, assemblies, methods, devices, apparatuses, combinations, etc. that may be utilized for repairing a valve, such as the mitral valve or tricuspid valve. Various features and steps as described elsewhere in this disclosure may be included in the examples summarized here. The methods, operations, steps, etc. described herein can be performed on a living animal or on a non-living cadaver, cadaver heart, simulator (e.g. with the body parts, tissue, etc. being simulated), etc.

In some applications, a system or an apparatus is provided that comprises an implant structure comprising an annuloplasty structure or annuloplasty ring structure that is configured to fit all or many different annulus sizes. Various systems, apparatuses, and methods are described which enable minimally invasive and/or transluminal (e.g., transcatheter, transvascular, etc.) positioning of the annuloplasty structure along the annulus without having to measure the size of the annulus in advance. In such a manner, systems and apparatuses described herein can accommodate and fits all annulus sizes of any patient or most annulus sizes of most patients.

The implant structure can comprise at least part of an annuloplasty structure (e.g., an annuloplasty ring structure, a partial annuloplasty ring, a full annuloplasty ring, annuloplasty band, etc.) for repairing a dilated valve annulus of a native atrioventricular valve, such as a mitral or tricuspid valve, of a patient.

For some applications, an elongate annuloplasty structure, such as that described above, is transluminally positioned along the annulus and is sequentially attached (e.g., anchored, fastened, clipped, adhered, etc.) thereto. In such applications, the elongate annuloplasty structure can be longer than needed for a given annulus, and thus, the elongate annuloplasty structure comprises apparatus or features which enables shortening of the overall length of the elongate annuloplasty structure while maintaining the body portion of the annuloplasty structure intact (e.g., by not cutting or severing any portion of the body portion). For some applications, the elongate annuloplasty structure is delivered to the annulus in the elongate state, attached (e.g., anchored, etc.) in part to the annulus, and then subsequently to the attaching, shortened. For some applications, the elongate annuloplasty structure is shortened (e.g., compressed) in conjunction with the anchoring process, e.g., alternating between shortening and attaching.

For some applications, the annuloplasty structure is controllably expandable, in part. For such applications, the annuloplasty structure is delivered to the annulus in the shortened state, expanded in part, and subsequently, attached (e.g., anchored, etc.) to the annulus. For some applications, the expanding of the annuloplasty structure occurs in conjunction with the attaching. For example, in such applications, the annuloplasty structure comprises a plurality of attachment sections or anchoring sections alternately disposed with a plurality of controllably-expandable sections. The controllably-expandable sections are controllably expandable by one or more control wires.

There is therefore provided, in accordance with some applications, a system or an apparatus, including an annuloplasty structure. The annuloplasty structure including a body portion (such as an annuloplasty ring body portion, an annuloplasty body portion, etc.) including flexible material. In some applications, the body portion is shaped and/or configured to define one or more controllably-expandable sections, each one of the one or more controllably-expandable sections having a respective first length. In some applications, the body portion includes one or more control wires coupled to the one or more controllably-expandable sections, the one or more control wires being movable to facilitate expansion each one of the one or more controllably-expandable sections to assume a respective second length that is greater than the respective first length. The system, apparatus, and/or annuloplasty structure also includes a contracting member and/or a contracting mechanism or attachment mechanism comprising and/or coupled to the contracting member. The contracting member is coupled to the body portion and is configured to contract the body portion independently of the one or more control wires.

In some applications, each one of the one or more controllably-expandable sections includes a part of the flexible material of the body portion, the part being folded, and wherein the one or more control wires is threaded through folds of the part.

In some applications, the part is folded telescopically in a direction away from a longitudinal axis of the body portion at the part.

In some applications, the part is folded in a manner in which at least one pouch is formed between layers of folds of the part, and the pouch has a longitudinal length that is measured along a pouch axis that is generally parallel with a longitudinal axis of the body portion at the part.

In some applications, the one or more control wires is removably coupled to the one or more controllably-expandable sections, and the flexible material is biased such that the one or more controllably-expandable sections expand upon decoupling of the one or more control wires from the one or more controllably-expandable sections.

In some applications, the one or more control wires is fixedly attached to the one or more controllably-expandable sections, and the one or more control wires facilitates expansion of the one or more controllably-expandable sections responsively to pulling on the one or more control wires.

In some applications, the one or more controllably-expandable sections include a plurality of controllably-expandable sections.

In some applications, the one or more control wires includes exactly one control wire which controls all of the plurality of controllably-expandable sections.

In some applications, the one or more control wires includes a plurality of control wires, each one of the plurality of control wires being movable to facilitate expansion of a respective one of the plurality of controllably-expandable sections.

There is additionally provided, in accordance with some applications, a method, including delivering to an annulus of a heart valve of a patient an annuloplasty structure. The annuloplasty structure can be the same as or similar to the annuloplasty structures described above or elsewhere herein. For example, in some applications, the annuloplasty structure includes a body portion (e.g., an annuloplasty ring body portion, etc.) including flexible material, the body portion being shaped and/or configured to define one or more controllably-expandable sections, each one of the one or more controllably-expandable sections having a respective first length. The annuloplasty structure includes one or more control wires coupled to the one or more controllably-expandable sections, the one or more control wires being movable to expand each of the one or more controllably-expandable sections to assume a respective second length that is greater than the respective first length. The annuloplasty structure includes a contracting member and/or a contracting mechanism or attachment mechanism comprising and/or coupled to the contracting member, the contracting member coupled to the body portion and being configured to contract the body portion independently of the one or more control wires. The method further includes controllably expanding the one or more controllably-expandable sections by moving the one or more control wires. This method can be performed on a living animal or on a non-living cadaver, cadaver heart, simulator, anthropomorphic ghost, etc.

In some applications the one or more control wires is removably coupled to the one or more controllably-expandable sections, the flexible material is biased such that the one or more controllably-expandable sections expands upon removal of the one or more control wires, and controllably expanding includes decoupling the one or more control wires from the one or more controllably-expandable sections. In some applications, the one or more control wires is fixedly attached to the one or more controllably-expandable sections, and controllably expanding includes pulling on the one or more control wires.

In some applications, the one or more control wires includes exactly one control wire which controls all of the plurality of controllably-expandable sections, and controllably expanding the one or more controllably-expandable sections includes controllably expanding the plurality of controllably-expandable sections by gradually moving the exactly one control wire.

There is also provided, in accordance with some applications, a method, including delivering to an annulus of a heart valve of a patient an annuloplasty structure including a body portion including flexible material, the body portion being shaped and/or configured to define first and second controllably-expandable sections, each one of the first and second controllably-expandable sections having a respective first length. The method includes controllably expanding at least the first controllably-expandable section to a second length that is greater than the first length, and fixing the second controllably-expandable section to maintain the first length and prevent the second controllably-expandable section from expanding. This method can be performed on a living animal or on a non-living cadaver, cadaver heart, simulator, anthropomorphic ghost, etc.

In some applications, fixing the second controllably-expandable section includes driving a tissue anchor through material of the second controllably-expandable section.

In some applications, the method further includes, subsequently to the fixing of the second controllably-expandable section, contracting the body portion.

Each one of the first and second controllably-expandable sections can include a respective part of the flexible material of the body portion, the part being folded.

There is further provided, in accordance with some applications, a method, including delivering to an annulus of a heart valve of a patient an annuloplasty structure including a body portion. The body portion can include a flexible material. In some implementations, the method includes using an anchor-delivery system, anchoring a first section of the body portion to tissue of the annulus using a first tissue anchor. The method can also include subsequently to the anchoring, using the anchor-delivery system, compressing a second section of the body portion toward the first section of the body portion. The method can also include subsequently to the compressing of the second section, using the anchor-delivery system, driving a second tissue anchor through the body portion and into tissue of the annulus, and by driving the second tissue anchor, maintaining the compression of the second section of the body portion. This method can be performed on a living animal or on a non-living cadaver, cadaver heart, simulator, anthropomorphic ghost, etc.

In some applications, compressing the second section of the body portion includes reversibly engaging the anchor-delivery system with the second section.

In some applications, driving the second tissue anchor through the body portion includes driving the second tissue anchor through the second section of the body portion.

In some applications, subsequently to the maintaining of the compression of the second section, contracting the body portion using a contracting member coupled to the flexible material of the body portion.

There is also provided, in accordance with some applications, a system and/or an apparatus, including an annuloplasty structure (e.g., an annuloplasty ring structure, etc.) including a body portion (e.g., an annuloplasty ring body portion, etc.). In some applications, the body portion is shaped and/or configured to define a plurality of compressible sections, each one of the plurality of compressible sections having a first thickness. The body portion can also be shaped and/or configured to include a plurality anchor-designated sections, each one of the plurality of anchor-designated sections having a second thickness that is greater than the first thickness of the respective one of the plurality of compressible sections. The plurality anchor-designated sections can be disposed alternatingly with the plurality of compressible sections, each anchor-designated section being designated for delivery of a respective tissue anchor therethrough. In some applications, a contracting member is coupled to the body portion, the contracting member being configured to contract the body portion. The plurality of compressible sections of the flexible material can facilitate a reduction in overall compressive forces on the flexible material of the body portion during contracting of the body portion by the contracting member.

In some applications, the first thickness is 0.05-0.1 mm. In some applications, the second thickness is 0.15-0.2 mm. In some applications, the body portion includes a fabric.

In some applications, each one of the plurality of compressible sections is shaped and/or configured so as to define a window.

In some applications, the body portion includes at least one flexible material, the at least one flexible material defining the plurality of compressible sections and the plurality of anchor-designated sections.

In some applications, the body portion includes:
at least one first flexible material defining the plurality of compressible sections; and at least one second flexible material defining the plurality of anchor-designated sections.

In some applications, each one of the plurality of compressible sections is shaped and/or configured so as to define a compressible-section wall, and wherein the compressible-section wall has the first thickness.

In some applications, each one of the plurality of anchor-designated sections is shaped and/or configured so as to define an anchor-designated wall, and the anchor-designated wall has the second thickness.

There is also provided, in accordance with some applications, a system and/or an apparatus, including an annuloplasty structure (e.g., an annuloplasty ring structure, etc.). The annuloplasty structure can be the same as or similar to those described above or elsewhere herein. For example, in some applications, the annuloplasty structure includes a body portion (e.g., an annuloplasty ring body portion, etc.) having first and second body portion ends and including flexible material. The flexible material of the body portion can be shaped and/or configured to define an anchor-coupling region or attachment region between the first body portion end and a vicinity of the body portion between the first and second body portion ends, exclusive of the second body portion end. In some applications, the flexible material of the body portion is also shaped and/or configured to define a deformable region between the second body portion end and a vicinity of the body portion between the first and second body portion ends, exclusive of the first body portion end. The deformable region can be deformable in an absence of force applied thereto, and be deformable in order to shorten an overall length of the body portion.

In some applications, the system and/or apparatus further includes a contracting member coupled to the body portion, the contracting member being configured to contract at least the anchor-coupling region or attachment region of the body portion.

In some applications, the deformable region is deformable into a spiral configuration in the absence of the force applied to the deformable region.

In some applications, the deformable region includes nitinol, and the nitinol is configured to facilitate deforming of the deformable region.

In some applications, the deformable region assumes a linear configuration when a tube is disposed through at least a lumen of the deformable region.

In some applications, the tube includes an anchor-delivery system.

There is additionally provided, in accordance with some applications, a method, including delivering to an annulus of a heart valve of a patient an annuloplasty structure (e.g., an annuloplasty ring structure, etc.). The annuloplasty structure can be the same as or similar to other annuloplasty structures described above or elsewhere herein. For example, in some application, the annuloplasty structure includes a body portion (e.g., an annuloplasty ring body portion, etc.) having first and second body portion ends and including flexible material. In some applications, the flexible material of the body portion is shaped and/or configured to define an anchor-coupling region or attachment region between the first body portion end and a vicinity of the body portion between the first and second body portion ends, exclusive of the second body portion end. In some applications, the flexible material of the body portion is shaped and/or configured to define a deformable region between the second body portion end and a vicinity of the body portion between the first and second body portion ends, exclusive of the first body portion end. The deformable region can be deformable in an absence of force applied thereto, and can be deformable in order to shorten an overall length of the body portion. This method can be performed on a living animal or on a non-living cadaver, cadaver heart, simulator, anthropomorphic ghost, etc.

In some applications, the method further includes anchoring to the annulus the anchor-coupling region or attachment region while the deformable region assumes a first shape. The method can include subsequently to the anchoring, allowing the deformable region to deform and assume a second shape in the absence of force applied thereto.

In some applications, the method further includes contracting the body portion subsequently to the allowing the deformable region to deform.

There is yet additionally provided, in accordance with some applications, a system and/or an apparatus, including an annuloplasty structure (e.g., an annuloplasty ring structure, etc.). The annuloplasty structure can be the same as or similar to other annuloplasty structures described above or elsewhere herein. For example, the annuloplasty structure can include a body portion (e.g., an annuloplasty ring body portion, etc.) including flexible material including a plurality of interwoven fibers. The body portion has a longitudinal axis.

In some applications, the body portion has at least a first longitudinal section including a first portion of the plurality of interwoven fibers oriented at an angle of 20 - 70 degrees with respect to the longitudinal axis of the body portion. In some applications, the body portion also has a second portion of the plurality of interwoven fibers oriented at an angle of 110 - 160 degrees with respect to the longitudinal axis of the body portion.

In some applications, the body portion has at least a second longitudinal section adjacent the first longitudinal section, the second longitudinal section including:
a third portion of the plurality of interwoven fibers oriented at an angle of 0 - 15 degrees with respect to the longitudinal axis of the body portion; and
a fourth portion of the plurality of interwoven fibers oriented at an angle of 75 - 90 degrees with respect to the longitudinal axis of the body portion.

In some applications, the first longitudinal section has a compressibility that is greater than a compressibility of the second longitudinal section.

In some applications, the system and/or apparatus further includes at least one tissue anchor, and the second longitudinal section is designated for coupling thereto the at least one tissue anchor.

There is further provided, in accordance with some applications, a method, including delivering to an annulus of a heart valve of a patient an annuloplasty structure (e.g., an annuloplasty ring structure, etc.) including a body portion having opposite first and second end portions. In some applications, the first end portion has a greatest cross-sectional measurement that is greater than a greatest cross-sectional measurement of the second end portion. The method includes attaching (e.g., anchoring, etc.) the annuloplasty structure to the annulus. Subsequently to the attaching, the method can include folding the second end portion toward the first end portion. This method can be performed on a living animal or on a non-living cadaver, cadaver heart, simulator (e.g., with the annulus being a simulated annulus, etc.), anthropomorphic ghost, etc.

In some applications, the body portion is shaped and/or configured so as to define a lumen, and folding the second end portion includes pushing the second end portion into the lumen toward the first end portion.

In some applications, the greatest cross-sectional measurement of the first end portion is 4 to 6 mm.

In some applications, the greatest cross-sectional measurement of the second end portion is 3.0 to 3.9 mm.

There is also provided, in accordance with some applications, a method, including delivering to an annulus of a heart valve of a patient an annuloplasty structure (e.g., an annuloplasty ring structure, etc.). The annuloplasty structure can be similar to other annuloplasty structures described herein. In some applications, the annuloplasty structure includes a body portion (e.g., an annuloplasty ring body portion, etc.), which can comprise flexible material, and the body portion has a first longitudinal length during the delivering, and a contracting member coupled to the body portion, the contracting member being configured to contract the body portion. In some applications, the method also includes subsequently to the delivering, shortening at least a portion of the body portion to a second longitudinal length shorter than the first longitudinal length, independently of the contracting member, while maintaining the entirety of the body portion intact, and subsequently to the shortening, remodeling the annulus by contracting the body portion using the contracting member. In some applications, the method further includes anchoring the body portion to the annulus. This method can be performed on a living animal or on a non-living cadaver, cadaver heart, simulator, anthropomorphic ghost, etc.

In some applications, shortening the portion of the body portion includes shortening the portion of the body portion prior to the anchoring. In some applications, shortening the portion of the body portion includes shortening the portion of the body portion incrementally with the anchoring by anchoring a first section of the body portion to the annulus by driving an anchor through the first section using an anchor-delivery system, and subsequently, using the anchor-delivery system, compressing a second section of the body portion toward the first section of the body portion.

In some applications, shortening the portion of the body portion includes shortening the portion of the body portion subsequently to the anchoring.

In some applications, shortening the portion of the body portion subsequently to the anchoring includes allowing a deformable region of the body portion to assume a deformed shape.

In some applications, shortening the portion of the body portion includes folding a second end portion of the body portion toward a first end portion of the body portion.

In some applications, the body portion is shaped and/or configured so as to define a lumen, and folding includes pushing the second end portion into the lumen toward the first end portion.

There is also provided, in accordance with some applications, a system and/or an apparatus, including an annuloplasty structure (e.g., an annuloplasty ring structure, etc.). The annuloplasty structure including a body portion (e.g., an annuloplasty ring body portion, etc.). The body portion can comprise a first flexible material.

In some implementations, the body portion is shaped and/or configured to define a plurality of windows in the body portion or in flexible material of the body portion. In some implementations, the body portion is shaped and/or configured to define a plurality anchor-designated sections of the body portion or in flexible material of the body portion. The plurality of anchor-designated sections can be disposed alternatingly with the plurality of windows, each anchor-designated section being designated for delivery of a respective tissue anchor therethrough.

The system/apparatus can also include a plurality of covers including a second flexible material, the each one of the plurality of covers covering a respective one of the plurality of windows. The system/apparatus can also include a contracting member coupled to the body portion and to the plurality of covers, the contracting member being configured to contract the body portion, the plurality of windows in the flexible material facilitating a reduction in overall compressible forces on the flexible material of the body portion along the body portion during contracting of the body portion by the contracting member.

In some applications, each one of the plurality of windows is shaped and/or configured so as to define a longest dimension of 12-16 mm.

In some applications, the first flexible material has a first thickness, and wherein the second flexible member has a second thickness, the first thickness being greater than the second thickness.

In some applications, the first thickness is 0.05-0.1 mm. In some applications, the second thickness is 0.15-0.2 mm.

In some applications, the second flexible material includes:
a first portion of the plurality of interwoven fibers oriented at an angle of 20 - 70 degrees with respect to the longitudinal axis of the body portion; and
a second portion of the plurality of interwoven fibers oriented at an angle of 110 - 160 degrees with respect to the longitudinal axis of the body portion.

In some applications, the first flexible material includes:
a third portion of the plurality of interwoven fibers oriented at an angle of 0 - 15 degrees with respect to the longitudinal axis of the body portion; and
a fourth portion of the plurality of interwoven fibers oriented at an angle of 75 - 90 degrees with respect to the longitudinal axis of the body portion.

There is also provided, in accordance with some applications, a method, including delivering to an annulus of a heart valve of a patient an annuloplasty structure (e.g., an annuloplasty ring, annuloplasty ring structure, etc.) including a body portion (e.g., an annuloplasty ring body portion, etc.). The body portion can comprise a first flexible material. In some applications, the body portion is shaped and/or configured to define a plurality of windows in the flexible material; and a plurality anchor-designated sections of the flexible material, the plurality of anchor-designated sections being disposed alternatingly with the plurality of windows, each anchor-designated section being designated for delivery of a respective tissue anchor therethrough. The annuloplasty structure can further include a plurality of covers including a second flexible material, the each one of the plurality of covers covering a respective one of the plurality of windows. In some applications, a contracting member is coupled to the body portion, the contracting member being configured to contract the body portion, the plurality of windows in the flexible material facilitating a reduction in overall compressible forces on the flexible material of the body portion during contracting of the body portion by the contracting member. The method can further include contracting the body portion using the contracting member. This method can be performed on a living animal or on a non-living cadaver, cadaver heart, simulator, anthropomorphic ghost, etc.

There is additionally provided, in accordance with some applications, a system and/or apparatus, including an annuloplasty structure (e.g., an annuloplasty ring structure, etc.) having a longitudinal axis. The annuloplasty structure including a primary body portion. In some implementations, the annuloplasty structure also includes one or more linking segments removably coupled to the primary body portion.

In some implementations, the annuloplasty structure also includes a first coupling coupled to a first end of the primary body portion, and a second coupling coupled to a first end of a first linking segment of the one or more linking segments that is disposed adjacent to the first coupling of the first end of the primary body portion, the first and second couplings being shaped and/or configured so as to mate with each other.

In some implementations, the annuloplasty structure also includes a decoupling-prevention element extending longitudinally along at least the first and second couplings in order to prevent decoupling of the first and second couplings. Removal of the decoupling-prevention element from at least the first and second couplings can facilitate decoupling of the first and second couplings.

In some applications, the main body portion is flexible. In some applications, the one or more linking segments is flexible.

In some applications, the system and/or apparatus further includes an elongate contracting member extending along a longitudinal length of the primary body portion and along the one or more linking segments.

In some applications, the decoupling-prevention element includes an elongate contracting member extending along a longitudinal length of the primary body portion and the one or more linking segments.

In some applications, the system and/or apparatus further includes a contracting mechanism, coupled to the implant structure and configured to contract at least a portion of the implant structure.

In some applications:
the first coupling is shaped and/or configured so as to define a first lumen and includes one or more radially-moveable projections, the one or more radially-moveable projections being radially moveable in response to a force applied thereto, and
the second coupling is shaped and/or configured so as to define a second lumen and a negative space shaped so as to receive the one or more radially-moveable projections.

In some applications:
the first coupling is shaped and/or configured so as to define a first lumen and includes one or more radially-moveable projections, the one or more radially-moveable projections being biased to collapse radially inwardly in an absence of force applied thereto;
the second coupling is shaped and/or configured so as to define a second lumen and a negative space shaped and/or configured so as to receive the one or more radially-moveable projections; and
the decoupling-prevention element includes an elongate tube configured to maintain the one or more radially-moveable projections within the negative space of the second coupling in order to prevent decoupling of the first and second couplings while a portion of the elongate tube passes through the first and second lumens of the respective first and second couplings.

In some applications, the elongate tube includes a portion of an anchor-delivery system, and the anchor-delivery system is configured to deploy a tissue anchor through the one or more linking segments.

In some applications:
the one or more linking segments includes at least the first linking segment and at least a second linking segment,
the first linking segment includes a third coupling coupled to a second of the first linking segment,
the second linking segment includes a fourth coupling coupled to a first end of the second linking segment that is disposed adjacent to the first linking segment, the fourth coupling being shaped and/or configured so as to mate with the third coupling,
the decoupling-prevention element extends longitudinally at least through the third and fourth couplings in order to prevent decoupling of the third and fourth couplings, and
removal of the decoupling-prevention element from at least the third and fourth couplings facilitates decoupling of the third and fourth couplings.

In some applications:
the third coupling is shaped and/or configured so as to define a third lumen and includes one or more radially-moveable projections, the one or more radially-moveable projections being biased to collapse radially inwardly in an absence of force applied thereto;
the fourth coupling is shaped and/or configured so as to define a fourth lumen and a negative space shaped and/or configured so as to receive the one or more radially-moveable projections;
the elongate tube of the decoupling-prevention element is configured to maintain the one or more radially-moveable projections of the third coupling within the negative space of the fourth coupling in order to prevent decoupling of the third and fourth couplings while a portion of the elongate tube passes through the third and fourth lumens of the respective third and fourth couplings.

In some applications, the elongate tube includes a portion of an anchor-delivery system, and the anchor-delivery system is configured to deploy a tissue anchor through the second linking segments.

There is also provided, in accordance with some applications, a method, including delivering into a heart of a patient an annuloplasty structure (e.g., an annuloplasty ring structure, etc.) having a longitudinal axis. The annuloplasty structure includes a primary body portion. In some implementations, the annuloplasty structure also includes one or more linking segments removably coupled to the primary body portion. In some implementations, the annuloplasty structure also includes a first coupling coupled to a first end of the primary body portion and a second coupling coupled to a first end of a first linking segment of the one or more linking segments that is disposed adjacent to the first coupling of the first end of the primary body portion. The first and second couplings can be shaped and/or configured so as to mate with each other. The annuloplasty structure can also include a decoupling-prevention element extending longitudinally along at least the first and second couplings in order to prevent decoupling of the first and second couplings. In some implementations, the method further includes facilitating decoupling of the first and second couplings by removing the decoupling-prevention element from at least the first and second couplings. This method can be performed on a living animal or on a non-living cadaver, cadaver heart, simulator, anthropomorphic ghost, etc.

In some applications, the method further includes attaching (e.g., anchoring, etc.) the primary body portion to cardiac tissue prior to the facilitating of the decoupling. In some applications, the method further includes contracting at least the primary body portion.

In some applications, facilitating the decoupling of the first and second couplings includes applying the force to the one or more radially-moveable projections. In some applications, applying the force to the one or more radially-moveable projections includes applying a pulling force to the one or more linking segments.

In some applications, removing the decoupling-prevention element includes allowing the radially-movable projections to move out of the negative space of the second coupling.

There is additionally provided, in accordance with some applications, a system and/or apparatus, including an annuloplasty structure. The annuloplasty structure including a body portion. In some implementations, the body portion is shaped and/or configured to define a first segment having a first degree of stretchability, and a second segment having a second degree of stretchability that is greater than the first degree of stretchability. The annuloplasty structure can also include a contracting member coupled to the body portion, the contracting member being configured to contract the body portion, the body portion facilitating a reduction in overall compressive forces on the flexible material of the body portion during contracting of the body portion by the contracting member.

In some applications, the second segment is disposed contiguously with the first segment. In some applications, the first segment has an elasticity that is different from an elasticity of the second segment.

In some applications, the first segment is configured for implantation at an annulus of a cardiac valve of a patient between a vicinity of an anterolateral commissure and P2, and the second segment is configured for implantation between P2 and a posterolateral commissure.

In some applications, the first segment has a greatest first diameter, and the second segment has a second diameter that is smaller than the first diameter following stretching of the second segment.

There is yet additionally provided, in accordance with some applications, a method, including delivering to an annulus of a heart valve of a patient an annuloplasty structure. The annuloplasty structure includes a body portion. In some implementations, the body portion is shaped and/or configured to define a first segment having a first degree of stretchability, and a second segment having a second degree of stretchability that is greater than the first degree of stretchability. In some implementations, the annuloplasty structure also includes a contracting member coupled to the body portion, the contracting member being configured to contract the body portion, the body portion facilitating a reduction in overall compressive forces on the flexible material of the body portion during contracting of the body portion by the contracting member. The method can also include fixing the first segment to the annulus. The method can also include subsequently to the fixing of the first segment, stretching the second segment; and subsequently, fixing the second segment to the annulus. This method can be performed on a living animal or on a non-living cadaver, cadaver heart, simulator, ghost, etc.

In some applications, fixing the first segment includes driving a tissue anchor through material of the first segment.

In some applications, fixing the second segment includes driving a tissue anchor through material of the second segment.

In some applications, the method further includes, subsequently to the fixing of the second segment, contracting the body portion.

In some applications, the first segment has a first diameter, and stretching the second segment includes stretching the second segment to a second diameter that is smaller than the first diameter.

There is further provided, in accordance with some applications, a method, including delivering to an annulus of a heart valve of a patient an annuloplasty structure including a body portion. The method can also include driving a first tissue anchor through the body portion of the annuloplasty structure. The method can also include positioning a first section of the body portion along the annulus, the first section of the body portion having a first length. In some implementations, the method also includes fixing the first section of the body portion to the annulus by driving a second tissue anchor at a first distance from the first tissue anchor. The method can also include exposing a second section of the body portion, the second section having a second length that is smaller than the first length of the first section of the body portion, and stretching the second section of the body portion to a stretched state of the second section. The stretched state of the second section can have a third length that is greater than the second length of the second section prior to the stretching. The method can also include positioning the second section of the body portion in the stretched state along the annulus, and fixing the second section of the body portion in the stretched state to the annulus by driving a third tissue anchor at a second distance from the second tissue anchor that is equal to the first distance. This method can be performed on a living animal or on a non-living cadaver, cadaver heart, simulator (e.g., with the annulus being a simulated annulus, etc.), anthropomorphic ghost, etc.

In some applications, the method further includes prior to the positioning of the first section of the body portion along the annulus, releasing the first section of the body portion from a delivery tool, and exposing the second section of the body portion includes releasing the second section of the body portion from the delivery system.

In some applications, the first section has a first degree of stretchability, and the second section has a second degree of stretchability that is greater than the first degree of stretchability.

In some applications, the method further includes, subsequently to the fixing of the second section, contracting the body portion.

There is yet further provided, in accordance with some applications, a system and/or apparatus, including an annuloplasty structure including a body portion. In some implementations, the body portion is shaped and/or configured to define a first segment, and at least one telescoping second segment being configured to be disposed at least in part within a lumen of the first segment, the at least one telescoping second segment being moveable proximally and distally along a longitudinal axis of the body portion.

The annuloplasty structure can also include a contracting member coupled to the body portion, the contracting member being configured to contract the body portion, the body portion facilitating a reduction in overall compressive forces on the flexible material of the body portion during contracting of the body portion by the contracting member.

In some applications, the second segment is disposed contiguously with the first segment. In some applications, the first segment is configured for implantation at an annulus of a cardiac valve of a patient between a vicinity of an anterolateral commissure and P2, and the second segment is configured for implantation between P2 and a posterolateral commissure.

In some applications, the at least one telescoping second segment includes at least first and second telescoping segments disposed contiguously, the second telescoping segment being configured to be disposed at least in part within a lumen of the first telescoping segment, the second telescoping second segment being moveable proximally and distally along a longitudinal axis of the body portion.

In some applications, the first segment includes a first coupling element, and the at least one telescoping second segment includes a second coupling element, and the first and second coupling elements are configured to be coupled together following movement of the at least one telescoping second segment with respect to the first segment.

In some applications, the first segment and the at least one telescoping second segment are shaped and/or configured so as to define respective lumens, and the first and second coupling elements are ring-shaped.

There is also provided, in accordance with some applications, a method, including delivering to an annulus of a heart valve of a patient an annuloplasty structure. The annuloplasty structure including a body portion. The body portion can be shaped and/or configured to define a first segment and at least one telescoping second segment. In some implementations, the at least one telescoping second segment is configured to be disposed at least in part within a lumen of the first segment, the at least one telescoping second segment being moveable proximally and distally along a longitudinal axis of the body portion. The annuloplasty structure can also include a contracting member coupled to the body portion, the contracting member being configured to contract the body portion, the body portion facilitating a reduction in overall compressive forces on the flexible material of the body portion during contracting of the body portion by the contracting member. The method can also include fixing the first segment to the annulus. In some implementations, subsequently to the fixing of the first segment, the method includes moving the at least one telescoping second segment along the longitudinal axis, and subsequently, fixing the at least one telescoping second segment to the annulus. This method can be performed on a living animal or on a non-living cadaver, cadaver heart, simulator (e.g., with the annulus being simulated, etc.), anthropomorphic ghost, etc.

In some applications, fixing the first segment includes driving a tissue anchor through material of the first segment.

In some applications, fixing the at least one telescoping second segment includes driving a tissue anchor through material of the at least one telescoping second segment.

In some applications, the method further includes, subsequently to the fixing of the at least one telescoping second segment, contracting the body portion.

In some applications, the first segment includes a first coupling element, and the at least one telescoping second segment includes a second coupling element, and the method further includes coupling together the first and second coupling elements following the moving of the at least one telescoping second segment.

In some applications, the first segment and the at least one telescoping second segment are shaped and/or configured so as to define respective lumens, and the first and second coupling elements are ring-shaped.

There is further provided, in accordance with some applications, a system and/or apparatus for use with tissue of a heart of a subject, the system and/or apparatus including an elongate annuloplasty structure. The elongate annuloplasty structure can include a body portion (which can be flexible) that includes a wall. The wall can be a tubular wall that defines a lumen along the body portion.

In some implementations, the wall includes a sleeve and a helical member, stiffer than the sleeve, and coupled to the sleeve. The helical member can be configured to define a plurality of helical turns extending in a helical path along and around the lumen.

In some implementations, the system and/or apparatus includes a plurality of anchors, each of the anchors including a head and a tissue-engaging element. These can be dimensioned to be advanceable along a lumen or the lumen of the wall.

The system and/or apparatus can include at least one anchor driver. The anchor driver can be configured to, for each of the anchors: reversibly couple the head, advance the anchor along the lumen, and/or use the anchor to secure a respective helical turn to the tissue by driving the tissue-engaging element of the anchor, from the lumen, through the wall, and into the tissue, such that the helical turn becomes sandwiched between the head and the tissue.

In some applications, the tissue-engaging element of each of the anchors is configured to pierce the helical member, and the at least one anchor driver is configured to, for each of the anchors, use the anchor to secure the respective helical turn to the tissue by driving the tissue-engaging element of the anchor from the lumen, through the helical member at the helical turn, and into the tissue, such that the helical turn becomes sandwiched between the head and the tissue.

In some applications, the at least one anchor driver is configured to, for each of the anchors, use the anchor to secure the respective helical turn to the tissue by driving the tissue-engaging element of the anchor from the lumen, through the wall adj acent to the helical turn, and into the tissue, such that the helical turn becomes sandwiched between the head and the tissue.

In some applications, the at least one anchor driver is configured to intracorporeally advance each of the anchors into the lumen and along the lumen.

In some applications, the sleeve is elastic.

In some applications, each of the anchors is dimensioned to be advanceable through the lumen while the tissue-engaging element of the anchor is disposed distally to the head of the anchor.

In some applications, the helical member includes a fabric. In some applications, the helical member includes a polymer. In some applications, the helical member is metallic. In some applications, the helical member includes a shape memory material.

In some applications, the system and/or apparatus further includes an elongate contracting member extending along and coupled to the body portion, the contracting member being configured to contract the body portion upon being tensioned.

In some applications, the contracting member extends through the lumen, radially inward from the helical member.

In some applications, the system and/or apparatus further includes an actuatable adjustment mechanism configured to, upon actuation, contract the body portion by tensioning the contracting member. In some implementations, the actuatable adjustment mechanism is coupled to the body portion and to the contracting member.

In some applications, the system and/or apparatus further includes a tool that includes an anchor channel that extends into the lumen, and the anchor driver is configured to advance the anchor along the lumen by advancing the anchor through the anchor channel.

In some applications, the anchor channel has a distal opening, and the anchor driver is configured to advance the anchor out of a distal opening of the anchor channel and through a part of the wall that is directly in front of the distal opening.

In some applications, the tool is configured to transluminally deliver the annuloplasty structure to the heart while the anchor channel is disposed within the lumen.

In some applications, the tool is configured to progressively advance the body portion off of the anchor channel.

In some applications, the tool includes a protrusion that extends radially outward from the anchor channel, and engages the helical member, inhibiting the body portion from sliding off of the channel.

In some applications, the tool is configured to facilitate control of advancement of the body portion off of the anchor channel via revolution of the protrusion around a central longitudinal axis of the anchor channel.

In some applications, the protrusion is rigidly fixed to the anchor channel. In some applications, the protrusion is movable with respect to the anchor channel. In some applications, the protrusion is longitudinally slidable with respect to the anchor channel.

There is further provided, in accordance with some applications, a method, including transluminally advancing an elongate annuloplasty structure into a subject. The annuloplasty structure including a body portion (e.g., a flexible body portion) that has wall (e.g., a tubular wall, etc.). The wall can be a tubular wall that defines a lumen along the body portion.

In some implementations, the wall includes a sleeve, and a helical member, stiffer than the sleeve, and coupled to the sleeve. The helical member can be configured to define a plurality of helical turns extending in a helical path along and around the lumen; and

The method includes fastening the body portion to tissue of the subject. This can be done by using at least one anchor driver to advance a plurality of anchors along the lumen, each of the anchors including a head and a tissue-engaging element, and for each of the anchors, fastening a respective helical turn to the tissue by driving the tissue-engaging element of the anchor, from the lumen, through the wall, and into the tissue, such that the helical turn becomes sandwiched between the head and the tissue. This method can be performed on a living animal or on a non-living cadaver, cadaver heart, simulator (e.g., with the tissue being simulated tissue, etc.), anthropomorphic ghost, etc.

In some applications, using the anchor driver to fasten the respective helical turn includes using the anchor driver to fasten the respective helical turn to the tissue by driving the tissue-engaging element of the anchor, from the lumen, through the helical member at the helical turn, and into the tissue, such that the helical turn becomes sandwiched between the head and the tissue.

In some applications, using the anchor driver to fasten the respective helical turn includes using the anchor driver to fasten the respective helical turn to the tissue by driving the tissue-engaging element of the anchor, from the lumen, through the wall adjacent to the helical turn, and into the tissue, such that the helical turn becomes sandwiched between the head and the tissue.

In some applications, the method further includes intracorporeally advancing each of the anchors into the lumen.

In some applications, using the anchor driver to advance the plurality of anchors along the lumen includes using the anchor driver to advance the plurality of anchors along the lumen while the tissue-engaging element of the anchor is disposed distally to the head of the anchor.

In some applications, the annuloplasty structure further includes an elongate contracting member extending along and coupled to the body portion, and the method further includes contracting the body portion by tensioning the contracting member.

In some applications, the contracting member extends through the lumen, radially inward from the helical member, and contracting the body portion by tensioning the contracting member includes contracting the body portion by tensioning the contracting member that extends through the lumen, radially inward from the helical member.

The various systems and/or apparatuses described here and elsewhere herein can further include an adjustment mechanism (e.g., such as on the annuloplasty structure, a catheter, other device, etc.) and contracting the body portion by tensioning the contracting member includes contracting the body portion by tensioning the contracting member by actuating the adjustment mechanism. In some implementations, the adjustment mechanism is coupled to the body portion of the annuloplasty structure and/or to the contracting member. Further, methods herein can include contracting the body portion by tensioning the contracting member, including, for example, contracting the body portion by tensioning the contracting member by actuating the adjustment mechanism.

In some applications, the using an anchor driver to advance the plurality of anchors along the lumen includes using the anchor driver to advance the plurality of anchors through an anchor channel disposed within the lumen. In some applications, using the anchor driver to fasten the respective helical turn to the tissue includes using the anchor driver to fasten the respective helical turn to the tissue by advancing the anchor out of a distal opening of the anchor channel and through a part of the wall that is directly in front of the distal opening.

In some applications, transluminally advancing the annuloplasty structure includes transluminally delivering the annuloplasty structure into the subject while the anchor channel is disposed within the lumen.

In some applications, the method further includes, after fastening each of the helical turns to the tissue, advancing a part of the body portion off of the anchor channel.

In some applications, the anchor channel has a protrusion coupled thereto, extending radially outward from the anchor channel, and the method further includes controlling advancement of the body portion off of the anchor channel by revolving the protrusion around a central longitudinal axis of the anchor channel.

In some applications, revolving the protrusion around the central longitudinal axis of the anchor channel includes rotating the anchor channel around the central longitudinal axis of the anchor channel.

In some applications, controlling advancement of the body portion off of the anchor channel further includes longitudinally sliding the protrusion with respect to the anchor channel.

Other features and steps described elsewhere herein can also be used with the systems, apparatuses, devices, methods, etc. described above. A fuller understanding can be had from the following detailed description of embodiments thereof, taken together with the drawings, in which:

### BRIEF DESCRIPTION OF THE DRAWINGS

Figs. 1-5 and 6A-D are schematic illustrations of respective example annuloplasty structures which comprise controllably-expandable sections;
Fig. 7 is a schematic illustration of an example annuloplasty structure that can be made shorter following anchoring of the annuloplasty structure to tissue of the annulus, in accordance with some applications;
Figs. 8A-B are schematic illustrations of an example annuloplasty structure comprising a deformable section;
Figs. 9-12 are schematic illustrations of respective example annuloplasty structures which comprise respective fabric compositions;
Figs. 13A-C are schematic illustrations of an example system for compressing a portion of an annuloplasty structure;
Figs. 14-15 are respective flow charts of at least some example steps in respective techniques described herein with reference to Figs. 1-13C;
Fig. 16 is a schematic illustration of an example annuloplasty structure which comprises different fabric compositions;
Figs. 17A-F are schematic illustrations of an example annuloplasty structure comprising removable links;
Figs. 18A-C are schematic illustrations of an example system comprising a variably-stretchable annuloplasty ring structure;
Fig. 19 is a schematic illustration of an example system comprising a telescopically-expandable annuloplasty structure; and
Figs. 20, 21A-B, 22A-C, and 23 are schematic illustrations of an example annuloplasty system comprising an example elongate annuloplasty structure.

### DETAILED DESCRIPTION OF EMBODIMENTS

The following description and accompanying figures, which describe and show certain embodiments, are made to demonstrate, in a non-limiting manner, several possible configurations of systems, apparatuses, devices, methods, etc. that may be used for various aspects and features of the present disclosure. As one example, various systems, devices/apparatuses, and methods are described herein, including systems, platforms, devices, methods, etc. that relate to adjustable annuloplasty devices.

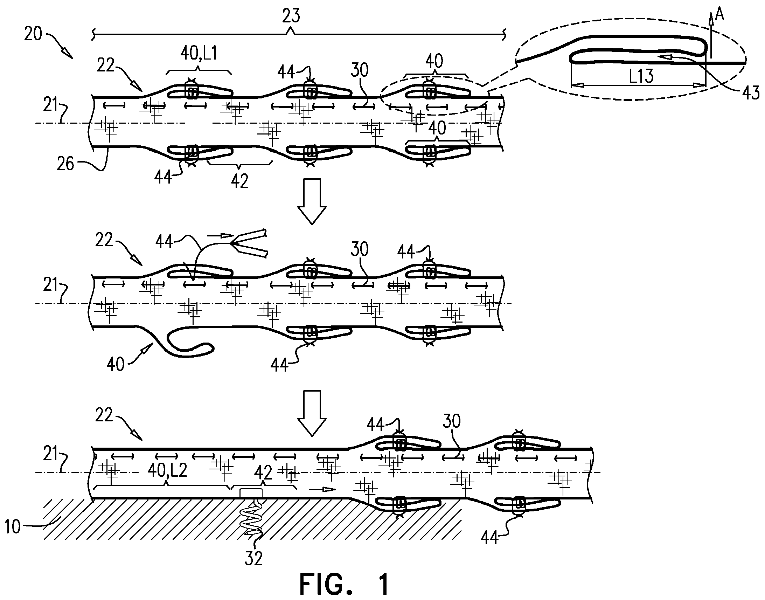

Fig. 1 is a schematic illustration of a system 20 comprising an implant comprising an annuloplasty structure 22 (e.g., an annuloplasty ring structure, an annuloplasty band, a partial annuloplasty ring, a full annuloplasty ring, etc.) which comprises a body portion 23 (e.g., an annuloplasty ring body portion) that is manufactured so that it is shaped and configured so as to define one or more controllably-expandable sections 40, in accordance with some applications. Annuloplasty structure 22 comprises a flexible sleeve 26 shaped and configured so as to define a lumen therethrough. Controllably-expandable sections 40 enable annuloplasty structure 22 to be delivered to an annulus of a cardiac valve of a patient (e.g., typically, but not necessarily a mitral valve or tricuspid valve) in a shortened state. Controllably-expandable sections 40 enable annuloplasty structure 22 to be controllably expanded once inside the body of the patient.

Each controllably-expandable section 40 can comprise a respective part of the flexible material of sleeve 26. During manufacture of sleeve 26, each section 40, which defines the part of sleeve 26 can be folded, or invaginated. Each of these parts can be folded telescopically in a direction A away from a longitudinal axis 21 of annuloplasty structure 22. This direction can be perpendicular to axis 21. Such telescopic folding enables compression of parts of sleeve 26 without affecting an overall inner diameter of sleeve 26, as shown in Fig. 1. Additionally, such telescopic folding enables the subsequent expansion of each section 40 along longitudinal axis 21.

Each of controllably-expandable sections 40, when in a compressed state, can define a longitudinal length L1 of 6-10 mm, e.g., 8 mm, measured longitudinal axis 21 of structure 22. When in an expanded state, each of controllably-expandable sections 40 can define a longitudinal length L2 of 12-18 mm, e.g., 16 mm, measured longitudinal axis 21 of structure 22. Longitudinal length L2 is greater than longitudinal length L1.

Each section 40 can be folded so as to form a pouch 43 between layers of folds of section 40. Pouch 43 can have a longitudinal length L13 of 6.5-9.5 mm, e.g., 7.5 mm, that is measured along a pouch axis that is generally parallel with a longitudinal axis of the annuloplasty ring body portion at the part of the annuloplasty ring body portion that comprises section 40.

Each fold of section 40 can optionally be held together by a knot 44, e.g., a slip knot or any other tie which can come apart easily. For some applications, sleeve 26 is manufactured such that sections 40 are biased such that each section 40 expands passively upon removal of knot 44. For some applications, as shown hereinbelow, each section 40 can be pulled open by a respective control wire.

For some applications, sleeve 26 is expanded once structure 22 is positioned against cardiac tissue.

As illustrated in the bottom figure in Fig. 1, once a given controllably-expandable section 40 is expanded, a tissue anchor 32 or other attachment means can be used to anchor or attach, to cardiac tissue, a section 42 of sleeve 26 adjacent to the now-expanded controllably-expandable section 40. While tissue anchor 32 is depicted in various embodiments and figures herein as a helical anchor that can be rotated or screwed into tissue, other types of tissue anchors or other attachment means can also be used (e.g., flexible anchors, rigid anchors, staples, fasteners, clips, adhesives, sutures, etc.). This sequential anchoring of sleeve 26 to the tissue immediately following the expansion of a first controllably-expandable section 40 but before expansion of a second controllably-expandable section 40 enables the operating physician alternate between expansion and anchoring and enables the operating physician to control the amount of overall expansion of sleeve 26. Once a sufficient amount of sleeve 26 is anchored to annulus tissue 10, the physician can choose to keep the remaining controllably-expandable sections 40 in their compressed states. In such a manner, system 20 minimizes the likelihood of any excess sleeve 26 interfering with the cardiac valve. Additionally, with system 20, the physician does not need to measure the size of the annulus prior to positioning annuloplasty ring structure 22. In such a manner, structure 22 accommodates and fits all native annulus sizes.

For some applications, each section 42 is labeled with a radiopaque marker. For some applications, each section 42 is designated as an anchor-designated section. In the illustrated application, structure 22 comprises a plurality of controllably-expandable sections 40 that are alternately disposed with a plurality of anchor-designated sections 42.

Sleeve 26 can comprise a braided fabric mesh, e.g., comprising polyethylene terephthalate, Dacron (TM), other materials, etc. Body portion 23 (e.g., sleeve 26) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system 20 can comprise an actuatable adjustment mechanism or contracting mechanism. The adjustment mechanism or contracting mechanism can be part of and/or coupled to one or more of the annuloplasty structure 22, a flexible elongate contracting member 30 that extends along sleeve 26, and/or another component of the system (e.g., a catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated, such as with polytetrafluoroethylene (PTFE) or another polymer. For some applications, contracting member 30 comprises a plurality of wires that are intertwined to form a rope structure.

The actuatable adjustment mechanism or contraction mechanism can be configured in a variety of ways. For some applications, for example, the actuatable adjustment mechanism comprises a spool or rotating member around which the contracting member can collect. For some applications, the actuatable adjustment mechanism comprises a tensioner that causes a tension force to be applied to the contraction member. For some applications, the actuatable adjustment mechanism comprises a gripper that grips the contraction member in a way that allows a user to apply a tension force to the contraction member. For some applications, the actuatable adjustment mechanism comprises a catheter or multiple catheters configured to interact to draw the contracting member proximally and/or tension it to cause contraction of the annuloplasty structure (e.g., one catheter gripping and allowing pulling of the contraction member, while another catheter provides a resisting force to the annuloplasty structure, etc.). Other applications are also possible.

Once the physician has positioned structure 22 along the annulus and has expanded any number of controllably-expandable sections 40, contracting member 30 is used to contract annuloplasty structure 22. For some applications, contracting member 30 is coupled to an actuatable contracting mechanism as described in US Patent Application Publication 2014/0309661 to Sheps et al., and/or in US Patent Application Publication 2015/0272734 to Sheps et al., which are incorporated herein by reference. Additionally, implantation of structure 22 can be done using any methods described in the `661 and/or `734 applications.

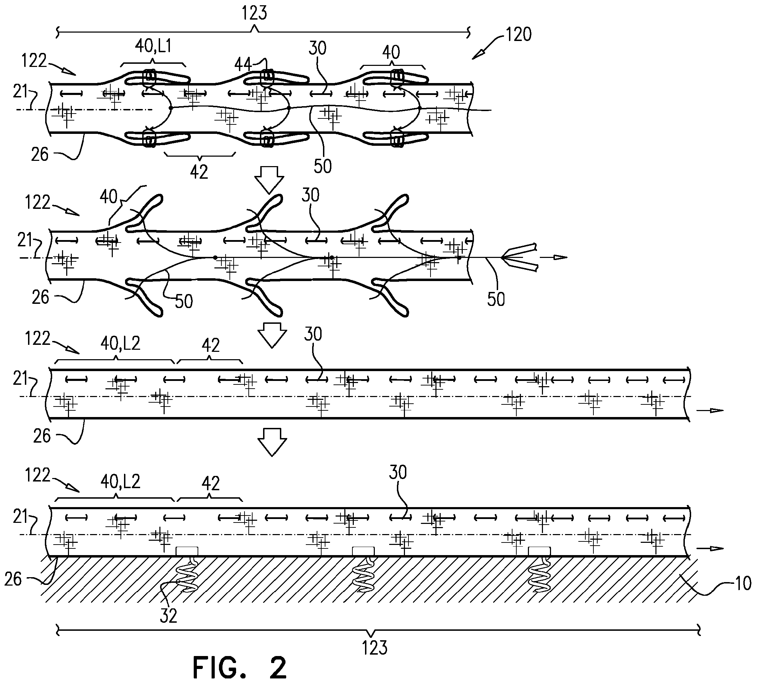

Figs. 2-3 are schematic illustrations of a system 120 comprising an implant comprising a structure 122 (e.g., an annuloplasty structure, an annuloplasty ring structure, etc.) which comprises body portion 123 (e.g., an annuloplasty body portion, an annuloplasty ring body portion, etc.) that is shaped and configured so as to define one or more controllably-expandable sections 40, in accordance with some applications. Except as described below, structure 122 is generally similar to structure 22, described hereinabove with reference to Fig. 1 and like reference numerals refer to like parts.

Structure 122 can comprise a single control wire 50 which is connected to all knots 44 and thereby to all controllably-expandable sections 40 (though multiple control wires can be used in some embodiments). In the illustrated embodiment, once control wire 50 is pulled, as shown, wire 50 releases all of knots 44. Once all of knots 44 are released, sleeve 26 can expand fully and be anchored to tissue. As shown in Fig. 2, once all controllably-expandable sections 40 have been expanded, anchors 32 are used to anchor sleeve 26 to the cardiac tissue. That is, only once sleeve 26 has been expanded, structure 122 is anchored to annulus tissue 10.

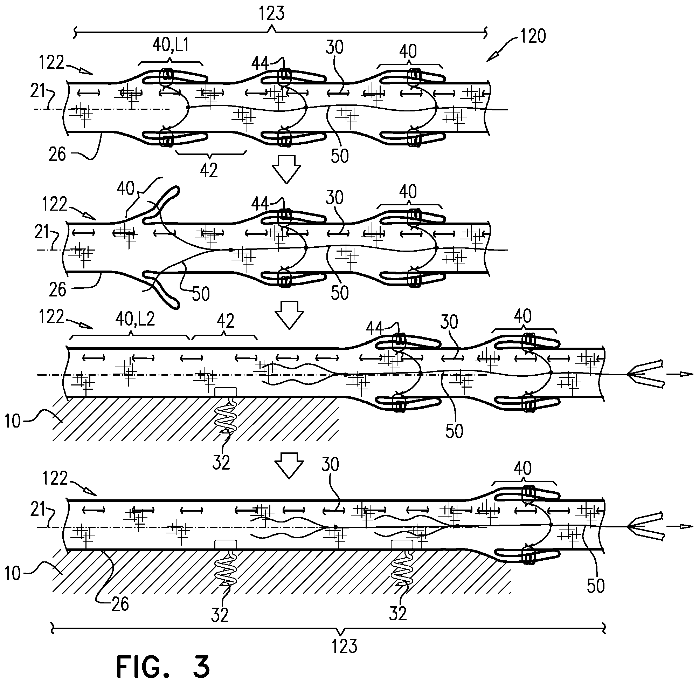

As shown in Fig. 3, control wire 50 can be pulled incrementally and gradually, and a respective anchor 32 or other attachment means can be used to anchor or attach sleeve 26 to the cardiac tissue. This sequential anchoring of sleeve 26 to the tissue immediately following the expansion of a first controllably-expandable section 40 but before expansion of a second controllably-expandable section 40 enables the operating physician alternate between expansion and anchoring and also enables the operating physician to control the amount of overall expansion of sleeve 26. Once a sufficient amount of sleeve 26 is anchored to annulus tissue 10, the physician can choose to keep the remaining controllably-expandable sections 40 in their compressed states. In such a manner, system 120 minimizes the likelihood of any excess sleeve 26 interfering with the cardiac valve. Additionally, with system 120, the physician does not need to measure the size of the annulus prior to positioning annuloplasty ring structure 122. In such a manner, structure 122 accommodates and fits all native annulus sizes.

Once the physician has positioned structure 122 along the annulus and has expanded any number of controllably-expandable sections 40, contracting member 30 is used to contract structure 122. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 122 can be done using any methods described in the `661 and/or `734 applications.

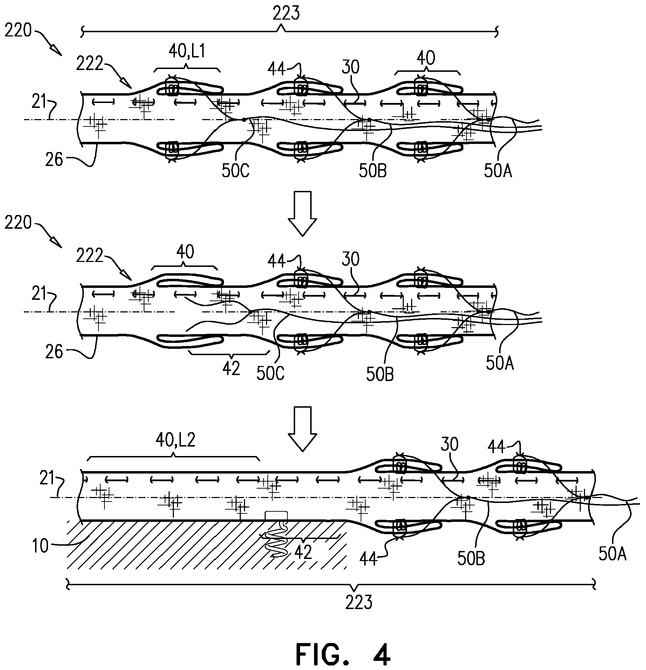

Fig. 4 is a schematic illustration of a system 220 comprising an implant comprising a structure 222 (e.g., an annuloplasty structure, an annuloplasty ring structure, etc.) which comprises a body portion 223 (e.g., an annuloplasty body portion, an annuloplasty ring body portion, etc.) that is shaped and configured so as to define one or more controllably-expandable sections 40, in accordance with some applications. Except as described below, structure 222 is generally similar to structure 22, described hereinabove with reference to Fig. 1 and like reference numerals refer to like parts.

Structure 222 can comprise a plurality of control wires, for example wires 50a, 50b, and 50c. That is, structure 222 can comprise a respective control wire 50 for each controllably-expandable section 40. Each of control wires 50a, 50b, and 50c can be coupled to knots 44 of a respective controllably-expandable section 40. Once each respective control wire 50 is pulled, as shown, wire 50 releases all of knots 44 of the respective controllably-expandable section.

In the illustrated application, once control wire 50a has been pulled, and section 40 expands, a tissue anchor 32 or other attachment means can be used to anchor or attach sleeve 26 to the cardiac tissue at section 42. This sequential anchoring of sleeve 26 to the tissue immediately following the expansion of a first controllably-expandable section 40, but before expansion of a second controllably-expandable section 40 by control wire 50b, enables the operating physician to alternate between expansion and anchoring and enables the operating physician to control the amount of overall expansion of sleeve 26. Once a sufficient amount of sleeve 26 is anchored to annulus tissue 10, the physician can choose to keep the remaining controllably-expandable sections 40 in their compressed states. In such a manner, system 20 minimizes the likelihood of any excess sleeve 26 interfering with the cardiac valve. Additionally, with system 20, the physician does not need to measure the size of the annulus prior to positioning annuloplasty ring structure 222. In such a manner, structure 222 accommodates and fits all native annulus sizes.

Once the physician has positioned structure 222 along the annulus and has expanded any number of controllably-expandable sections 40, contracting member 30 is used to contract annuloplasty structure 222 independently of any remaining control wires 50 coupled to the body portion of structure 222. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 222 can be done using any methods described in the `661 and/or `734 applications.

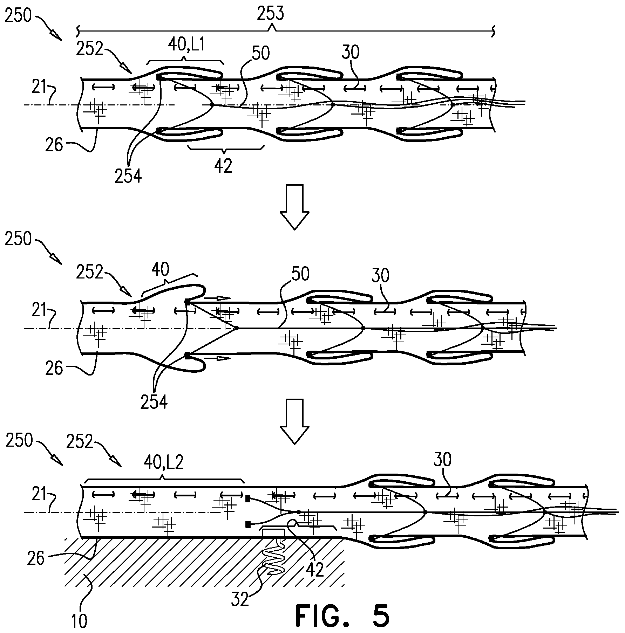

Fig. 5 is a schematic illustration of a system 250 comprising an implant comprising a structure 252 (e.g., an annuloplasty structure, an annuloplasty ring structure, etc.) which comprises a body portion (e.g., an annuloplasty body portion, an annuloplasty ring body portion, etc.) that is shaped and configured so as to define one or more controllably-expandable sections 40, in accordance with some applications. Except as described below, structure 252 is generally similar to structure 22, described hereinabove with reference to Fig. 1 and like reference numerals refer to like parts.

Structure 252 comprises a one or more control wires 50. For some applications, structure 252 comprises a respective control wire 50 for each controllably-expandable section 40. Respective portions of control wire 50 are reversibly coupled directly to material of sleeve 26 at controllably-expandable section 40, at coupling points 254. Once control wire 50 is pulled, as shown, wire 50 actively pulls on the material of sleeve 26 in order to facilitate expansion of controllably-expandable section 40.

As shown in the illustrated application, once control wire 50 has been pulled, and section 40 expands, a tissue anchor 32 or other attachment means can be used to anchor or attach sleeve 26 to the cardiac tissue at section 42. This sequential anchoring of sleeve 26 to the tissue immediately following the expansion of a first controllably-expandable section 40, but before expansion of a second controllably-expandable section 40 by control wire 50, enables the operating physician to alternate between expansion and anchoring and enables the operating physician to control the amount of overall expansion of sleeve 26. Once a sufficient amount of sleeve 26 is anchored to the annulus, the physician can choose to keep the remaining controllably-expandable sections 40 in their compressed states. In such a manner, system 250 minimizes the likelihood of any excess sleeve 26 interfering with the cardiac valve. Additionally, with system 250, the physician does not need to measure the size of the annulus prior to positioning annuloplasty ring structure 252. In such a manner, structure 252 accommodates and fits all native annulus sizes.

Once the physician has positioned structure 252 along the annulus and has expanded any number of controllably-expandable sections 40, contracting member 30 is used to contract annuloplasty structure 252 independently of any remaining control wires 50 coupled to the body portion of structure 252. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 252 can be done using any methods described in the `661 and/or `734 applications.

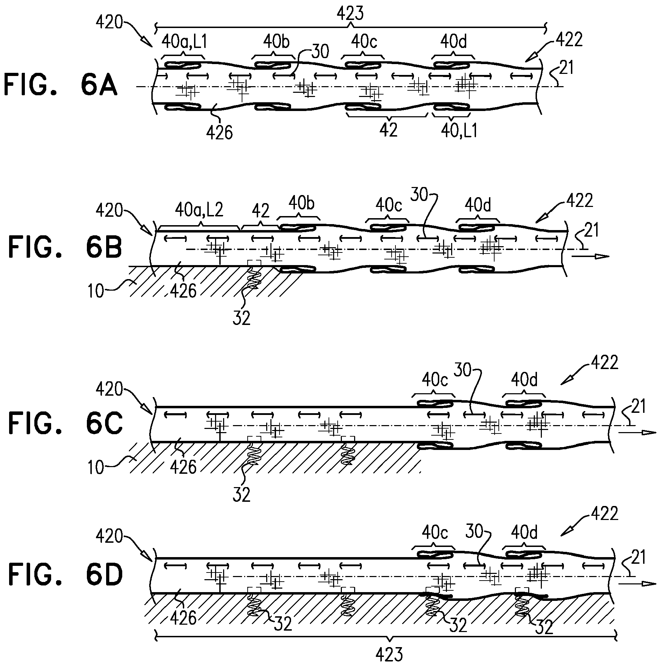

Figs. 6A-D are schematic illustrations of a system 420 comprising an implant comprising a structure 422 (e.g., an annuloplasty structure, an annuloplasty ring structure, etc.) which comprises a body portion (e.g., an annuloplasty body portion, an annuloplasty ring body portion, etc.) that is shaped and configured so as to define one or more controllably-expandable sections 40, in accordance with some applications. Structure 422 comprises a sleeve 426 and a contracting member 30. Except as described below, structure 422 is generally similar to structure 22, described hereinabove with reference to Fig. 1 and like reference numerals refer to like parts.

Sleeve 426 can be manufactured such that sections 40 are biased such that each section 40 retains its folded shape. For example, during manufacture, each section 40 can be ironed, or otherwise set, so as to assume the folded shape. For some applications, as shown hereinbelow, each section 40 can be pulled open by a respective control wire coupled thereto. For some applications, each section 40 can be pulled open by pulling on a different section, e.g., an end, of sleeve 426. Structure 422 is delivered to the annulus in a shortened state, as shown in Fig. 6A, in which controllably-expandable sections 40 assume their respective folded states, each section 40 assuming a length L1.

In Fig. 6B, once structure 422 is positioned along the annulus tissue 10, the physician pulls open a first section 40a and subsequently drives an anchor 32 into tissue 10 at anchor-designated section 42a, so as to retain the now-opened and unfolded state of section 40a, such that section 40a now assumes length L2 that is greater than length L1.

For some applications, each section 42 is labeled with a radiopaque marker. For some applications, each section 42 is designated as an anchor-designated section.

In Fig. 6C, the physician pulls open a second section 40b and subsequently drives an anchor 32 into annulus tissue 10 at anchor-designated section 42b, so as to retain the now-opened and unfolded state of section 40b, such that section 40b now assumes length L2 that is greater than length L1.

In Fig. 6D, the physician determines that the overall length of structure 422 anchored to annulus tissue 10 is sufficient and structure 422 therefore does not need to undergo any further expansion. In order to prevent inadvertent further expansion of sections 40c and 40d, the physician drives a respective tissue anchor 32 through each of sections 40c and 40d so as to retain sections 40c and 40d in their compressed states. Subsequently, contracting member 30 is used to contract structure 422 in order to remodel the shape of the annulus.

This sequential anchoring of sleeve 426 to the tissue immediately following the expansion of a first controllably-expandable section 40a but before expansion of a second controllably-expandable section 40b enables the operating physician to alternate between expansion and anchoring and enables the operating physician to control the amount of overall expansion of sleeve 426. Once a sufficient amount of sleeve 426 is anchored to the annulus, the physician can choose to keep the remaining controllably-expandable sections 40c and 40d in their compressed states. In such a manner, system 420 minimizes the likelihood of any excess sleeve 426 interfering with the cardiac valve. Additionally, with system 420, the physician does not need to measure the size of the annulus prior to positioning annuloplasty ring structure 422. In such a manner, structure 422 accommodates and fits all native annulus sizes.

Sleeve 426 can comprise a braided fabric mesh, e.g., comprising polyethylene terephthalate, Dacron (TM), other materials, etc. Body portion 23 (e.g., sleeve 26) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system 420 comprises an actuatable adjustment mechanism or contracting mechanism. The adjustment mechanism or contracting mechanism can be part of and/or coupled to one or more of the annuloplasty structure 422, a flexible elongate contracting member 30 that extends along sleeve 426, and/or another component of the system. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Once the physician has positioned structure 422 along the annulus and has expanded any number of controllably-expandable sections 40, contracting member 30 is used to contract annuloplasty structure 422. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 422 can be done using any methods described in the `661 and/or `734 applications.

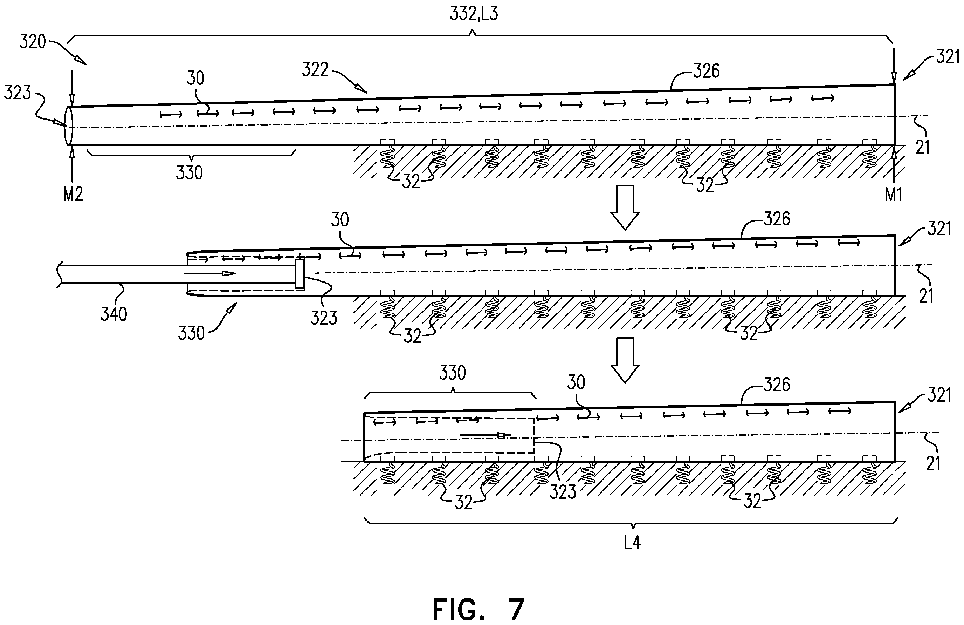

Fig. 7 is a schematic illustration of a system 320 comprising a structure 322 (e.g., an annuloplasty structure, an annuloplasty ring structure, etc.) having a flexible body portion 332 (e.g., an annuloplasty body portion, an annuloplasty ring body portion, etc.) comprising a tapered sleeve 326 and a contracting member 30, in accordance with some applications. Sleeve 326 is shaped and configured so as to define a lumen therethrough. Sleeve 326 is shaped and configured as to define a first end 321 having a first greatest cross-sectional measurement M1 of 4-6 mm, e.g., 5 mm, and a second end 323 having a second greatest cross-sectional measurement M2 of 3.0-3.9 mm, e.g., 3.5 mm, which is smaller than the first greatest cross-sectional measurement.

Any number of tissue anchors 32 or other attachment means can be used to anchor or attach structure 322 to the annulus of the valve. Once a sufficient amount of sleeve 326 is anchored to the annulus, the physician folds, or pushes, the excess portion 330 of sleeve 326 into the lumen of the portion of sleeve 326 that has already been anchored to the annulus, using a pushing tool 340. That is, the physician folds a second end portion of sleeve 326 toward a first end portion of sleeve 326. In such a manner, system 320 minimizes the likelihood of any excess sleeve 326 interfering with the cardiac valve. Additionally, with system 320, the physician does not need to measure the size of the annulus prior to positioning annuloplasty ring structure 322. In such a manner, structure 322 accommodates and fits all native annulus sizes.

For some applications a cutting tool (not shown) can be used to cut excess portion 330 of sleeve 326.

Sleeve 326 can comprise a braided fabric mesh, e.g., comprising polyethylene terephthalate, Dacron (TM), other materials, etc. Body portion 332 (e.g., sleeve 326) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system 320 comprises an actuatable adjustment mechanism or contracting mechanism. The adjustment mechanism or contracting mechanism can be part of and/or coupled to one or more of the annuloplasty structure 322, a flexible elongate contracting member 30 that extends along sleeve 326, and/or another component of the system (e.g., a catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Once the physician has positioned structure 322 along the annulus and has pushed excess portion 330 within the lumen of the portion of sleeve 326 already anchored to the cardiac tissue, contracting member 30 is used to contract annuloplasty structure 322. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 322 can be done using any methods described in the `661 and/or `734 applications.

In such a manner, system 320 facilitates delivering of an elongate structure or annuloplasty ring structure (e.g., structure 322, as shown) to the annulus in an elongate state, anchoring in part to the annulus, and then subsequently to the anchoring, shortening of the elongate structure while maintaining the body portion of the structure or annuloplasty ring structure intact (e.g., by not cutting or severing any portion of the body portion), and subsequently to the shortening, contracting of the structure or annuloplasty ring structure by contracting member 30 in order to contract and remodel the annulus of the patient. In such a manner, the pre-shortening of the structure or annuloplasty ring structure reduces the overall contraction force that is required in order to contract the structure or annuloplasty ring structure.

System 320, therefore, provides a method for (a) delivering to the annulus of the patient structure 322 while body portion 332 has a first longitudinal length L3 of 120-136 mm, (b) subsequently to the delivering, shortening body portion 332 to a second longitudinal length L4 of 40-68 mm independently of contracting member 30, while maintaining the entirety of annuloplasty body portion 332 intact (e.g., no part of portion 332 is cut or severed), and (c) subsequently to the shortening, remodeling the annulus by contracting body portion 332 using contracting member 30. This means that body portion 332 will only need to be contracted along a smaller length, i.e., length L4, rather than along a greater length L3, i.e., if excess portion 330 were not to be pushed within the lumen of sleeve 326. As such, pushing excess portion 330 within the lumen of sleeve 326 reduces the overall force needed to contract body portion 332 once anchored to the annulus.

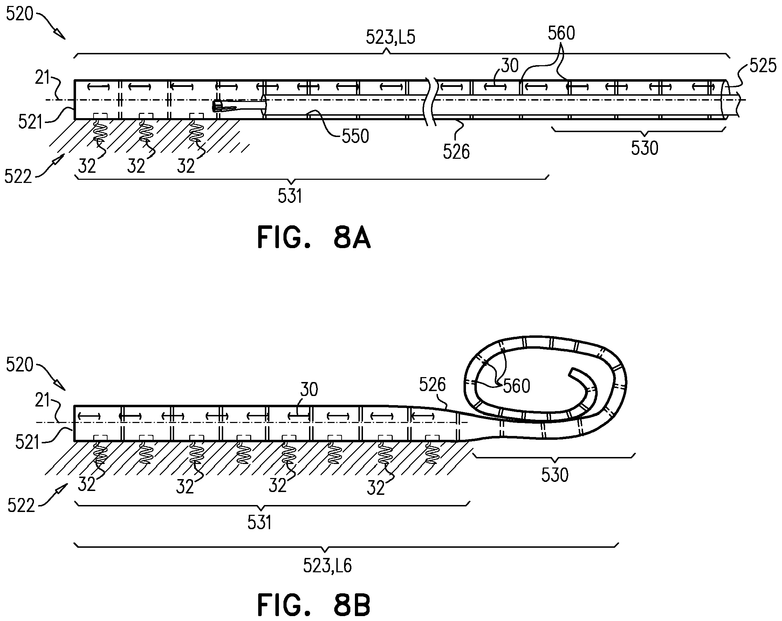

Figs. 8A-B are schematic illustrations of a system 520 comprising a structure 522 (e.g., an annuloplasty structure, an annuloplasty ring structure, etc.) having a flexible body portion 523 (e.g., an annuloplasty body portion, an annuloplasty ring body portion, etc.) comprising (a) a sleeve 526 having an anchor-coupling region or attachment region 531 and a deformable region 530 and (b) a contracting member 30, in accordance with some applications. Sleeve 526 is shaped and configured so as to define a lumen therethrough. Body portion 523 is shaped and configured as to define a first body portion end 521 and a second body portion end 525. Anchor-coupling region or attachment region 531 is disposed between first body portion end 521 and a vicinity of body portion 523 between first and second body portion ends 521 and 525, exclusive of second body portion end 525. Deformable region 530 is disposed between second body portion end 525 and a vicinity of body portion 523 between the first and second body portion ends 521 and 525, exclusive of first body portion end 521. Additionally, body portion 523 can comprise a plurality of radiopaque markers 560 which indicate to the physician where to implant anchors 32.

Optionally, deformable region 530 can comprise a super-elastic material, e.g., nitinol, which enables deformable region 530 to assume its shape. For some applications, the super-elastic material has shape-memory.

Any number of tissue anchors 32 or other attachment means can be used to anchor or attach structure 522 to the annulus of the valve using an anchor-delivery system or attachment system 550. In the illustrated application, anchor-delivery system 550 comprises a tube. While the tube of system 550 is disposed within at least the lumen of deformable region 530, deformable region 530 assumes a first, generally linear shape, as shown in Fig. 8A. Once a sufficient amount of sleeve 526 is anchored to the annulus, the physician removes system 550 from within the lumen of sleeve 526 and deformable region 530 passively assumes a second, deformed shape. As shown, the second, deformed shape is a spiral by way of illustration and not limitation. As such, with system 520, the physician does not need to measure the size of the annulus prior to positioning annuloplasty ring structure 522. In such a manner, structure 522 accommodates and fits all native annulus sizes.

Sleeve 526 can comprise a braided fabric mesh, e.g., comprising polyethylene terephthalate, Dacron (TM), other materials, etc. Body portion 523 (e.g., sleeve 526) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, system 520 comprises an actuatable adjustment mechanism or contracting mechanism. The adjustment mechanism or contracting mechanism can be part of and/or coupled to one or more of the annuloplasty structure 522, a flexible elongate contracting member 30 that extends along sleeve 526, and/or another component of the system (e.g., a catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Once the physician has positioned structure 522 along the annulus and allowed deformable region 530 to assume the deformed shape, contracting member 30 is used to contract annuloplasty structure 522. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 522 can be done using any methods described in the `661 and/or `734 applications.

In such a manner, system 520 facilitates delivering of an elongate structure or annuloplasty ring structure (e.g., structure 522, as shown) to the annulus in an elongate state, anchoring in part to the annulus, and then subsequently to the anchoring, shortening of the elongate structure while maintaining the body portion of the structure or annuloplasty ring structure intact (e.g., by not cutting or severing any portion of the body portion), and subsequently to the shortening, contracting of the structure or annuloplasty ring structure by contracting member 30 in order to contract and remodel the annulus of the patient. In such a manner, the pre-shortening of the structure or annuloplasty ring structure reduces the overall contraction force that is required in order to contract the structure or annuloplasty ring structure.

System 520, therefore, provides a method for (a) delivering to the annulus of the patient structure 522 while body portion 523 has a first longitudinal length L5 of 122-130 mm, e.g., 125 mm (b) subsequently to the delivering, shortening body portion 523 to a second longitudinal length L6 of 90-129 mm, e.g., 100 mm, independently of contracting member 30, while maintaining the entirety of body portion or annuloplasty body portion 523 intact (e.g., no part of portion 523 is cut or severed), and (c) subsequently to the shortening, remodeling the annulus by contracting body portion 523 using contracting member 30. This means that body portion 523 will only need to be contracted along a smaller length, i.e., length L6, rather than along a greater length L5, i.e., if deformable region were not to be deformed. As such, allowing region 530 to deform reduces the overall force needed to contract body portion 523 once anchored to the annulus.

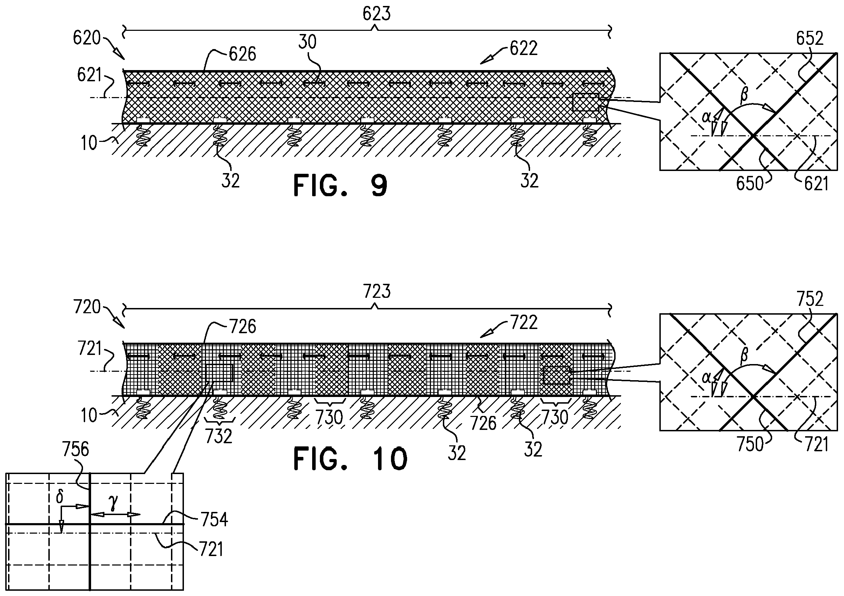

Reference is now made to Fig. 9, which is a schematic illustration of a system 620 comprising a structure 622 (e.g., an annuloplasty structure, an annuloplasty ring structure, etc.) having a flexible body portion 623 (e.g., annuloplasty body portion, an annuloplasty ring body portion, etc.) comprising a sleeve 626 and a contracting member 30, in accordance with some applications. Body portion 623 comprises a sleeve 626. Body portion 623 comprises flexible material comprising a plurality of interwoven fibers.

A first portion 650 of the plurality of interwoven fibers are oriented at an angle α (alpha) of 20 - 70 degrees, e.g., 45 degrees, with respect to a longitudinal axis 621 of body portion 623. A second portion 652 of the plurality of interwoven fibers are oriented at an angle β (beta) of 110 - 160 degrees, e.g., 135 degrees with respect to a longitudinal axis 621 of annuloplasty body portion 623. First and second portions 650 and 652 of interwoven fibers imparts increased longitudinal compressibility and/or stretchability to sleeve 626.

Sleeve 626 can comprise a braided fabric mesh, e.g., comprising polyethylene terephthalate, Dacron (TM), other materials, etc. Body portion 623 (e.g., sleeve 626) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 622, a flexible elongate contracting member 30 that extends along sleeve 626, and/or another component (e.g., catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Once the physician has positioned structure 622 along the annulus and has anchored or attached structure 622 to the annulus, contracting member 30 is used to contract annuloplasty structure 622. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 622 can be done using any methods described in the `661 and/or `734 applications.

Reference is now made to Fig. 10, which is a schematic illustration of a system 720 comprising a structure 722 (e.g., an annuloplasty structure, an annuloplasty ring structure, etc.) having a flexible body portion 723 (e.g., annuloplasty body portion, an annuloplasty ring body portion, etc.) comprising a sleeve 726 and a contracting member 30, in accordance with some applications. Body portion 723 comprises a sleeve 726. Body portion 723 comprises flexible material comprising first and second longitudinal sections 730 and 732 which alternate along a longitudinal length of body portion 723.

First longitudinal section 730 comprises flexible material comprising a plurality of interwoven fibers. A first portion 750 of the plurality of interwoven fibers are oriented at an angle α (alpha) of 20 - 70 degrees, e.g., 45 degrees, with respect to a longitudinal axis 721 of annuloplasty ring body portion 723. A second portion 752 of the plurality of interwoven fibers are oriented at an angle β (beta) of 110 - 160 degrees, e.g., 135 degrees with respect to a longitudinal axis 721 of annuloplasty ring body portion 723. First and second portions 750 and 752 of the interwoven fibers imparts increased longitudinal compressibility and/or stretchability to first longitudinal section 730.

Second longitudinal section 732 comprises flexible material comprising a plurality of interwoven fibers. A third portion 754 of the plurality of interwoven fibers are oriented at an angle γ (gamma) of 0 - 15 degrees, e.g., 0 degrees or 180 degrees, with respect to a longitudinal axis 721 of annuloplasty body portion 723. A fourth portion 756 of the plurality of interwoven fibers are oriented at an angle δ (delta) of 75 - 90 degrees, e.g., 90 degrees with respect to a longitudinal axis 721 of annuloplasty body portion 723. Third and fourth portions 754 and 756 of the interwoven fibers imparts increased strength, rigidity, and/or stability to second longitudinal section 732. Thus, each of second longitudinal sections 732 is designated for driving a respective tissue anchor 32 therethrough, i.e., sections 732 are anchor-designated sections.

Therefore, there is typically a trade-off between (i) strength, rigidity, and/or stability, and (ii) compressibility and/or stretchability. By providing both (i) anchor-designated sections 732 with increased strength, rigidity, and/or stability specifically where anchors will be anchored, and (ii) sections 730 with increased compressibility and/or stretchability specifically where anchors will not be anchored, structure 722 is provided with both (i) sufficient strength, rigidity, and/or stability for anchors 32 to be fastened thereto, and (ii) sufficient compressibility and/or stretchability for adapting to various annulus sizes.

Sleeve 726 can comprise a braided fabric mesh, e.g., comprising polyethylene terephthalate, Dacron (TM), other materials, etc. Body portion 723 (e.g., sleeve 726) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 722, a flexible elongate contracting member 30 that extends along sleeve 726, and/or another component (e.g., catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Once the physician has positioned structure 722 along the annulus and has anchored or attached structure 722 to the annulus, contracting member 30 is used to contract annuloplasty structure 722. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 722 can be done using any methods described in the `661 and/or `734 applications.

Thus, first longitudinal sections 730 have a greater degree of compressibility than second longitudinal sections 732. As such, first longitudinal sections 730 reduce the overall compressible force to body portion 723 by contracting member 30 during contraction of structure 722.

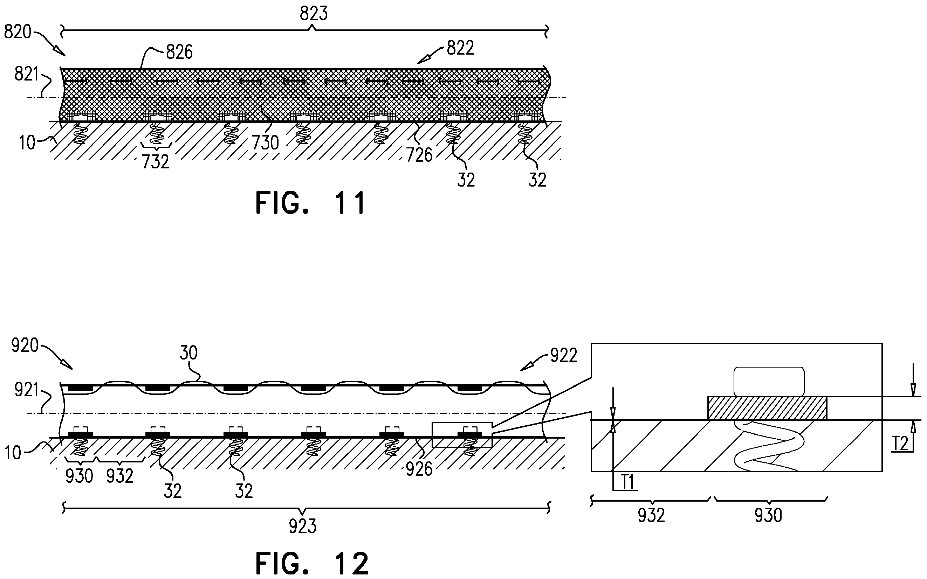

Reference is now made to Fig. 11, which is a schematic illustration of a system 820 comprising a structure 822 (e.g., an annuloplasty structure, an annuloplasty ring structure, etc.) having a flexible body portion (e.g., an annuloplasty body portion, an annuloplasty ring body portion, etc.) comprising a sleeve 826 and a contracting member 30, in accordance with some applications. Body portion 823 comprises flexible material comprising first and second longitudinal sections 730 and 732 which alternate along a longitudinal length of body portion 823 and along a longitudinal axis 821 of structure 822. Except as described below, structure 822 is generally similar to structure 722, described hereinabove with reference to Fig. 10 and like reference numerals refer to like parts.

As shown in Fig. 11, sleeve 826 defines second longitudinal sections 732 only at an area through which tissue anchor 32 is deployed, i.e., at a portion of the lateral wall of sleeve 826 designated to rest against annulus tissue 10. Sections 732 can be disposed opposite contracting member 30. Unlike in Fig. 10 which shows each one of sections 732 occupying 360 degrees of the circumference of sleeve 726, each section 732 of structure 822 of Fig. 11 occupies 75 - 180 degrees of the circumference of sleeve 826. In such a manner, a majority of sleeve 826 comprises sections 730, and thus, sleeve 826 exhibits a high degree of compressibility and/or expandability.

Once the physician has positioned structure 822 along the annulus and has anchored or attached structure 822 to the annulus, contracting member 30 is used to contract annuloplasty structure 822. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 822 can be done using any methods described in the `661 and/or `734 applications.

Reference is now made to Fig. 12, which is a schematic illustration of a system 920 comprising a structure 922 (e.g., an annuloplasty structure, an annuloplasty ring structure, etc.) having a flexible body portion 923 (e.g., an annuloplasty body portion, an annuloplasty ring body portion, etc.) comprising sleeve 926 and a contracting member 30, in accordance with some applications. Body portion 923 comprises flexible material comprising first and second longitudinal sections 932 and 930 which alternate along a longitudinal length of body portion 923 and along a longitudinal axis 921 of structure 922.

Each one of first sections 932 defines a compressible section having compressible-section wall having a first thickness T1 of 0.05-0.1 mm, e.g., 0.07 mm. Each one of second sections 930 defines an anchor-designated section having an anchor-designated wall having a second thickness T2 of 0.15-0.2 mm, e.g., 0.18 mm. Second thickness T2 is greater than first thickness T1 and second thickness T2 is such so as to impart rigidity and/or stability to the respective sections 930 of sleeve 926 though which a respective anchor 32 is deployed. For some applications, each section 930 is labeled with a radiopaque marker.

First longitudinal sections 932, due to their respective thickness, have a greater degree of compressibility than second longitudinal sections 930. As such, first longitudinal sections 932 reduce the overall compressible force to body portion 923 by contracting member 30 during contraction of structure 922.

Sleeve 926 can comprise a braided fabric mesh, e.g., comprising polyethylene terephthalate, Dacron (TM), other materials, etc. Body portion 923 (e.g., sleeve 926) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 922, a flexible elongate contracting member 30 that extends along sleeve 926, and/or another component (e.g., catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Once the physician has positioned structure 922 along the annulus and has anchored or attached structure 922 to the annulus, contracting member 30 is used to contract annuloplasty structure 922. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 922 can be done using any methods described in the `661 and/or `734 applications.

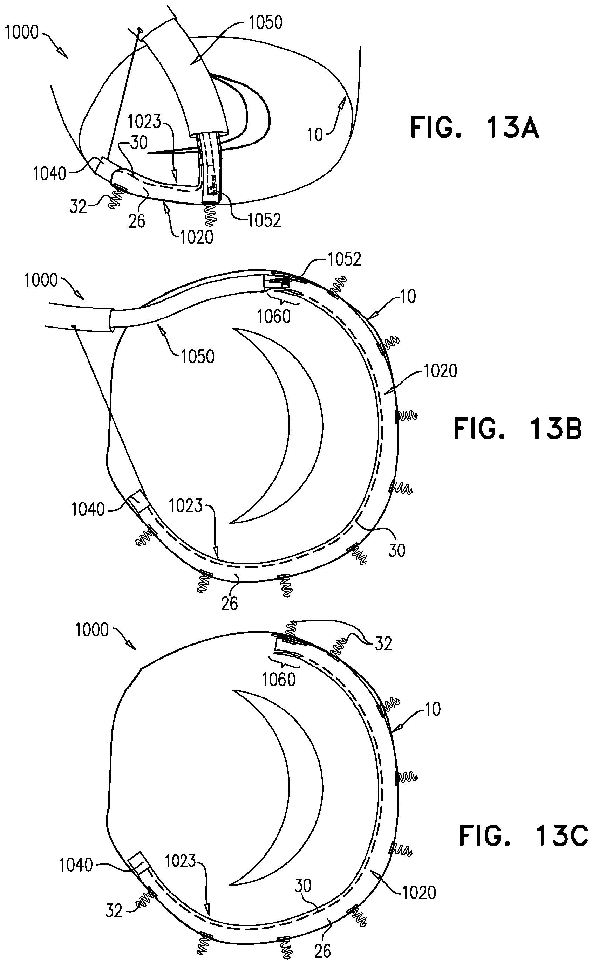

Reference is now made to Figs. 13A-C, which are schematic illustrations of a system 1000 comprising a structure 1020 (e.g., an annuloplasty structure, an annuloplasty ring structure, etc.) having a flexible body portion 1023 (e.g., an annuloplasty body portion, an annuloplasty ring body portion, etc.) comprising a sleeve 26 and a contracting member 30, in accordance with some applications. It is to be noted that annuloplasty structure 1020 can comprise any annuloplasty structure or annuloplasty ring structure described herein with reference to Figs. 1-12. Additionally, structure 1020 can comprise a contracting mechanism 1040 comprising and/or coupled to contracting member 30 as described in US Patent Application Publication 2014/0309661 to Sheps et al., and/or in US Patent Application Publication 2015/0272734 to Sheps et al., which are incorporated herein by reference. Additionally, implantation of structure 1020 can be done using any methods described in the `661 and/or `734 applications.

In Fig. 13A, an anchor-delivery system 1050 or other attachment system can be used to anchor or attach a first section of annuloplasty body portion 1023 of annuloplasty structure 1020, for example, by deploying one or more tissue anchors 32 through sleeve 26 and into annulus tissue 10, from within a lumen of sleeve 26.

In Fig. 13B, anchor-delivery system 1050 is used to compress a second section 1060 of annuloplasty body portion 1023 toward the first section of annuloplasty body portion 1023. For some applications, system 1050 can reversibly engage and be reversibly coupled to a portion of sleeve 26 during the compressing of second section 1060. For example, an anchor driver 1052 can reversibly ensnare a portion of sleeve 26.

In Fig. 13C, anchor-delivery system 1050 is used to drive a tissue anchor 32 through the now-compressed second section 1060 of body portion 1023. For some applications, the tissue anchor can be delivered downstream of section 1060. It is to be noted that Figs. 13A-C show compression of only a single section 1060 of body portion 1023 by way of illustration and not limitation. Sequential anchoring or attachment of sleeve 26 to the tissue can be done immediately or shortly following the compression respective sections of body portion 1023 which enables the operating physician alternate between compression and anchoring and also enables the operating physician to control the amount of overall compression of sleeve 26. In such a manner, system 1000 minimizes the likelihood of any excess sleeve 26 interfering with the cardiac valve. Additionally, with system 1000, the physician does not need to measure the size of the annulus prior to positioning annuloplasty structure 1020. In such a manner, structure 1020 accommodates and fits all native annulus sizes.

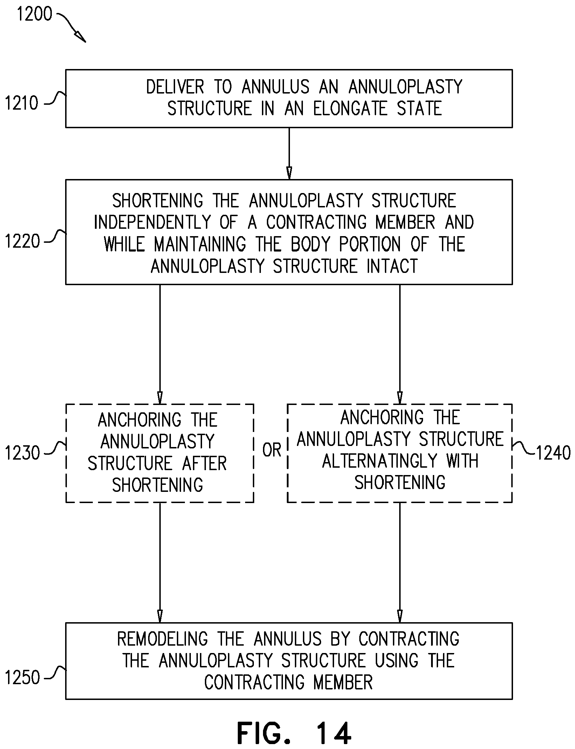

Figs. 14-15 are respective flow charts of at least some steps in respective techniques described herein with reference to Figs. 1-13C, in accordance with respective applications.

Fig. 14 shows a method 1200 for delivering a structure (e.g., an annuloplasty structure, an annuloplasty ring structure, etc.) in an elongate state and subsequently shortening the structure prior to contraction. In step 1210, a structure or "annuloplasty structure" (which can be any of the annuloplasty structures or similar structures described herein) is delivered to the annulus and/or heart atrium in an elongate state. In step 1220, the annuloplasty structure is shortened independently of contracting member 30, while maintaining the body portion of the annuloplasty structure intact (e.g., by not cutting or severing any portion of the body portion).

For some applications, the annuloplasty structure is anchored to the annulus tissue only once the structure has been shortened in accordance with the decision of the operating physician (step 1230). Optionally, the annuloplasty structure is shortened alternatingly with anchoring, as per step 1240. That means, the annuloplasty structure can be shortened in part, then anchored to the annulus tissue using a first tissue anchor. Subsequently, the annuloplasty structure can be shortened in part, again, then anchored to the annulus tissue using a second tissue anchor. Finally, in step 1250, the annulus is remodeled by contracting the annuloplasty structure using the contracting member 30.

Reference is now made to Figs. 7, 8, 13A-C, 14, and 17A-F. It is to be noted that method 1200 described herein with reference to Fig. 14 can be applied to systems and apparatuses described herein with reference to Figs. 7, 8, 13A-C, and 17A-F.

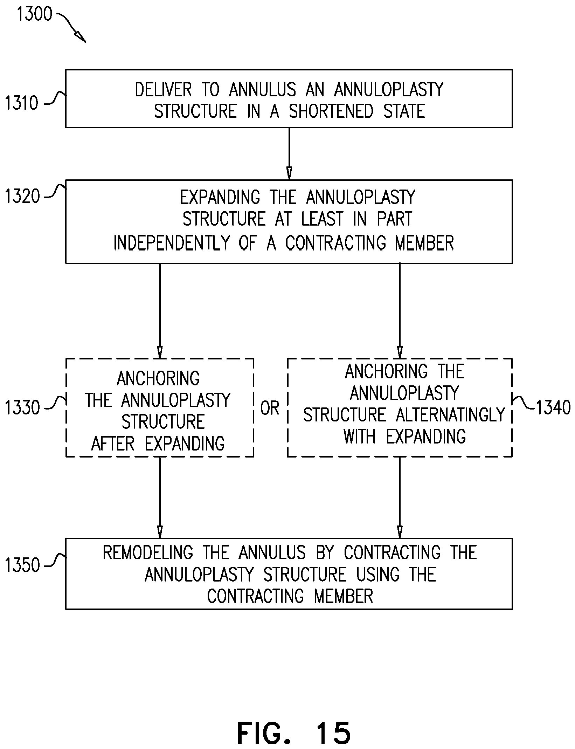

Fig. 15 shows a method 1300 for delivering a structure (e.g., an annuloplasty structure, an annuloplasty ring structure, etc.) in a shortened state and subsequently expanding the structure prior to contraction. In step 1310, an annuloplasty structure (which can be any of the annuloplasty structures or similar structures described herein) is delivered to the annulus and/or heart atrium in a shortened state. In step 1320, the annuloplasty structure is expanded at least in part independently of contracting member 30, while maintaining the body portion of the annuloplasty structure intact (e.g., by not cutting or severing any portion of the body portion).

For some applications, the annuloplasty structure is anchored to the annulus tissue only once the structure has been expanded in accordance with the decision of the operating physician (step 1330). Optionally, the annuloplasty structure is expanded alternatingly with anchoring, as per step 1340. That means, the annuloplasty structure can be expanded in part, then anchored to the annulus tissue using a first tissue anchor. Subsequently, the annuloplasty structure can be expanded in part, again, then anchored to the annulus tissue using a second tissue anchor. Finally, in step 1350, the annulus is remodeled by contracting the annuloplasty structure using the contracting member 30.

Reference is now made to Figs. 1-5, 6A-D, 15, 18A-C, and 19. It is to be noted that method 1200 described herein with reference to Fig. 15 can be applied to systems and apparatuses described herein with reference to Figs. 1-5, 6A-D, 18A-C, and 19.

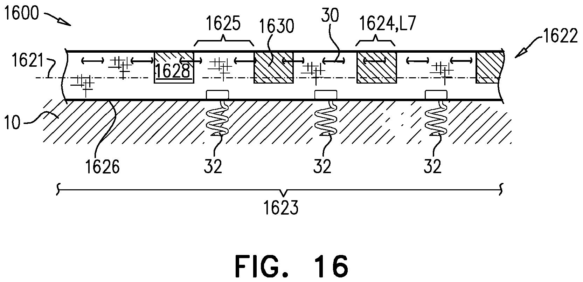

Reference is now made to Fig. 16, which is a schematic illustration of a system 1600 comprising a structure 1622 (e.g., an annuloplasty structure, an annuloplasty ring structure, etc.) having a flexible body portion 1623 (e.g., an annuloplasty body portion, an annuloplasty ring body portion, etc.) comprising sleeve 1626 and a contracting member 30, in accordance with some applications. Sleeve 1626 of body portion 1623 comprises a first flexible material and is shaped and configured so as to define a plurality of windows 1628 which each covered by a respective cover 1630 comprising a second, flexible material that is more stretchable and/or compressible than the first flexible material of sleeve 1626. Sleeve 1626 thereby defined a plurality of anchor-designated sections or attachment sections 1625 alternating along a longitudinal axis 1621 of structure 1622 with a plurality of compressible sections 1624 defined by windows 1628. As shown, a respective tissue anchor 32 can anchor structure 1622 to annulus tissue 10 at each anchor-designated section 1625.

In some applications, each window 1628, and each compressible section 1624 has a longest dimension L7 of 12-16 mm, e.g., 14 mm.

Reference is now made to Figs. 10 and 16. For some applications, each cover 1630 of Fig. 16 can comprise the same material of first longitudinal section 730 and can comprise flexible material comprising a plurality of interwoven fibers. First and second portions 750 and 752 of the interwoven fibers imparts increased longitudinal compressibility and/or stretchability to first longitudinal section 730, and thereby, to covers 1630 of Fig. 16. Each anchor-designated section 1625 can comprise the same material of second longitudinal section 732 which comprises flexible material comprising a plurality of interwoven fibers. Third and fourth portions 754 and 756 of the interwoven fibers imparts increased rigidity and/or stability to second longitudinal section 732, and thereby, to anchor-designated section 1625. For some applications, each section 1625 is labeled with a radiopaque marker.

Covers 1630, due to their respective interwoven fibers, have a greater degree of compressibility and/or stretchability than anchor-designated sections 1625. As such, windows 1628 and covers 1630 reduce the overall compressible force to body portion 1623 by contracting member 30 during contraction of structure 1622.

Reference is now made to Figs. 12 and 16. For some applications, each cover 1630 of Fig. 16 can comprise the same material of first section 932 of Fig. 12 which can define a compressible section having a first thickness T1 of 0.05-0.1 mm, e.g., 0.07 mm. Each anchor-designated section or attachment section 1625 can comprise the same material of second section 930 which can have a second thickness T2 of 0.15-0.2 mm, e.g., 0.18 mm. Second thickness T2 can be greater than first thickness T1 and second thickness T2 can be configured to impart rigidity and/or stability to the respective sections of sleeve 1626 though which a respective anchor is deployed. For some applications, each section 1625 is labeled with a radiopaque marker.

Covers 1630, due to their respective thickness, have a greater degree of compressibility than anchor-designated sections 1625. As such, windows 1628 and covers 1630 reduce the overall compressible force to body portion 1623 by contracting member 30 during contraction of structure 1622.

Reference is again made to Fig. 16. Sleeve 1626 can comprise a braided fabric mesh, e.g., comprising polyethylene terephthalate, Dacron (TM), other materials, etc. Body portion 1623 (e.g., sleeve 1626) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 1622, a flexible elongate contracting member 30 that extends along sleeve 1626, and/or another component. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Once the physician has positioned structure 1622 along the annulus and has anchored or attached structure 1622 to the annulus, contracting member 30 is used to contract annuloplasty structure 1622. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al., which are incorporated herein by reference. Additionally, implantation of structure 922 can be done using any methods described in the `661 and/or `734 applications.

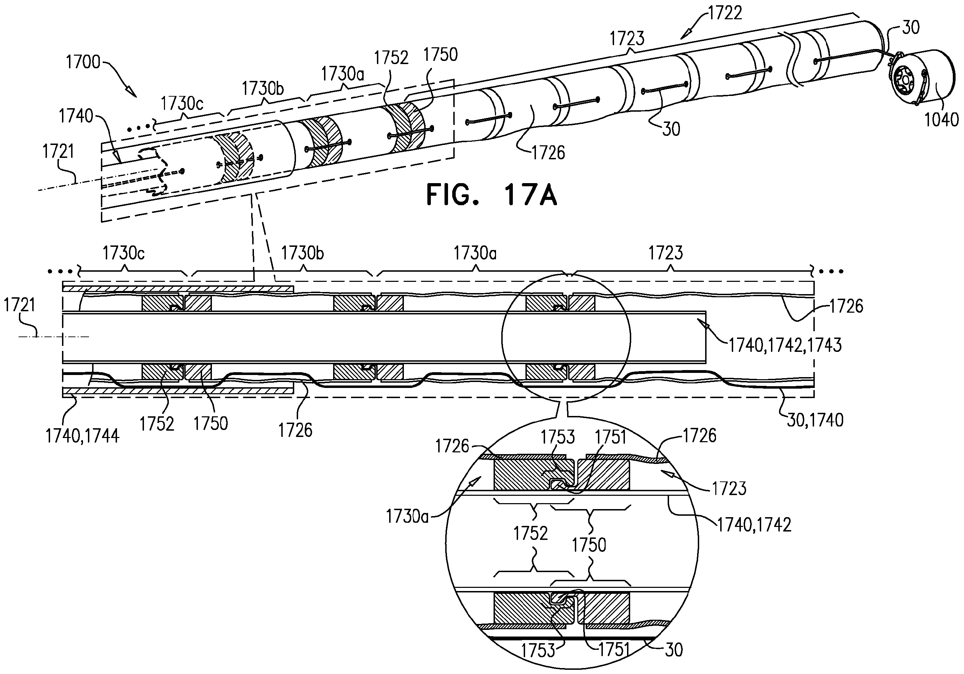

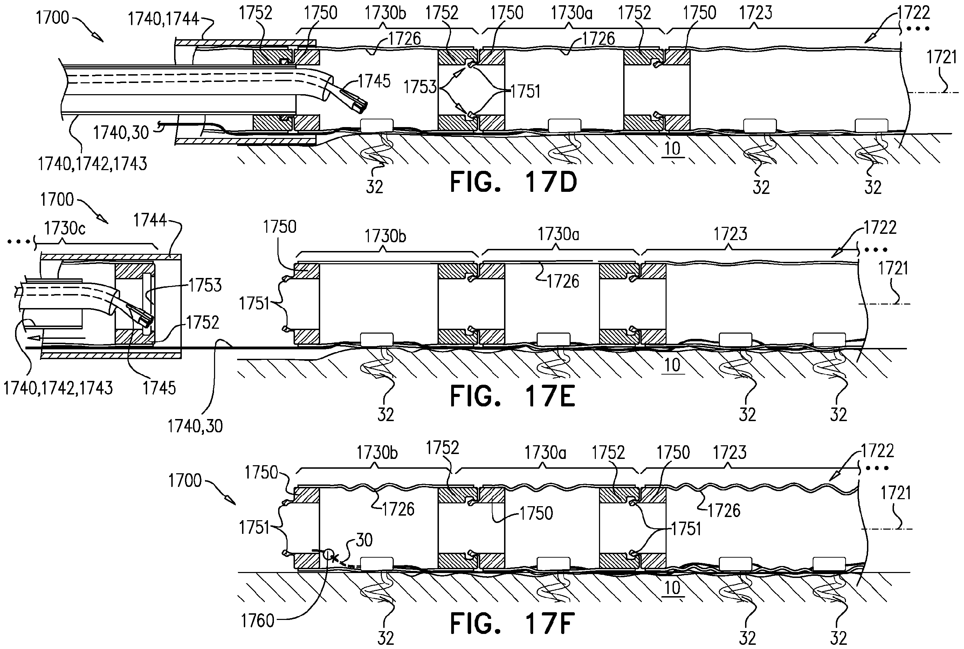

Reference is now made to Figs. 17A-F, which are schematic illustrations of a system 1700 comprising a structure 1722 (e.g., an annuloplasty structure, an annuloplasty ring structure, etc.) having a primary body portion 1723 comprising a flexible sleeve 1726 and a contracting member 30, in accordance with some applications. Sleeve 1726 of body portion 1723 comprises a flexible material shaped and configured so as to define a lumen therethrough. Structure 1722 comprises one or more linking segments 1730, e.g., 1730a, 1730b, and 1730c, as shown. Structure 1722 is shown as comprising three segments 1730 by way of illustration and not limitation. It is to be noted that structure 1722 can comprise any number of segments 1730. Segments 1730 are removably coupled to primary body portion 1723.

Structure 1722 is provided comprising any number of linking segments 1730 coupled to primary body portion 1723 such that structure 1722 can be delivered to the annulus of the patient in an elongate state and, subsequently, any number of linking segments 1730 can be decoupled from primary body portion 1723. Once a sufficient amount of structure 1722 (i.e., primary body portion 1723 and any number of linking segments 1730) is anchored to annulus tissue 10, the physician can choose to decouple and remove from the patient's body the remaining linking segments 1730. In such a manner, system 1700 minimizes the likelihood of any excess sleeve 1726 interfering with the cardiac valve. Additionally, with system 1700, the physician does not need to measure the size of the annulus prior to positioning annuloplasty ring structure 1722. In such a manner, structure 1722 accommodates and fits all native annulus sizes.

Primary body portion 1723 as well as linking segments 1730 comprise flexible sleeve 1726.

Sleeve 1726 can comprise a braided fabric mesh, e.g., comprising polyethylene terephthalate, e.g., Dacron (TM). Body portion 1723 (e.g., sleeve 1726) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 1722, a flexible elongate contracting member 30 that extends along sleeve 1726, and/or another component. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

Contracting member 30 extends along a longitudinal length of primary body portion 1723 as well as along each of linking segments 1730. Contracting member 30 can be used to contract primary body portion 1723 as well as linking segments 1730 along a longitudinal axis 1721 of structure 1722.

A first end, e.g., a proximal end of primary body portion 1723 can comprise a first coupling 1750. A second coupling 1752 can be coupled to a first end, e.g., a distal end, of a first linking segment 1730a that is disposed adjacent to first coupling 1750 of the first end of primary body portion 1723. First and second couplings 1750 and 1752 are shaped and configured so as to mate with each other.

A third coupling (e.g., another coupling 1750) can be coupled to a second end, e.g., a proximal end, of first linking segment 1730a. A fourth coupling (e.g., another coupling 1752) can be coupled to a first end (e.g., a distal end) of a second linking segment 1730b that is disposed adjacent to first linking segment 1730a.

First coupling 1750 is shaped and configured so as to define a first lumen and comprises one or more radially-moveable projections 1751. Second coupling 1752 is shaped and configured so as to define a second lumen and a negative space 1753 shaped and configured so as to receive one or more radially-moveable projections 1751.

For some applications, radially-moveable projection 1751 of first coupling 1750 comprises a single, circular projection and negative space 1753 of second coupling 1752 is shaped and configured so as to define a single, circular groove.

For some applications, radially-moveable projections 1751 are biased to collapse radially inwardly in an absence of force applied thereto. For some applications, radially-moveable projections 1751 are not biased to collapse radially inwardly, and are moveable in response to a force applied thereto. For example, the walls defining negative space 1753 of second coupling 1752 are shaped and configured so as to enable slidable decoupling of the one or more projections 1751 in one direction, in response to a force applied to linking segment 1730, e.g., a pulling force applied to linking segment 1730.

System 1700 comprises a decoupling-prevention element 1740, as shown in Fig. 17A. Decoupling-prevention element extends longitudinally along at least first and second couplings 1750 and 1752 in order to prevent decoupling of first and second couplings 1750 and 1752. During delivery of structure 1722, decoupling-prevention element 1740 extends along all of linking segments 1730 as well as along a portion of a primary body portion 1723 and prevents decoupling of linking segments 1730 from each other as well as from primary body portion 1723.

For some applications, decoupling-prevention element 1740 comprises an elongate tube 1742. The external surface of tube 1742 is sized so as to rest against or push against one or more projections 1751 so as to maintain projections 1751 within negative space 1753 and prevent decoupling of first and second couplings 1750 and 1752.

For applications in which one or more projections 1751 are biased to move radially inward, the external surface of tube 1742 of decoupling-prevention element 1740 pushes against one or more projections 1751 of first coupling 1750 and maintains projections 1751 within negative space 1753 of second coupling 1752 in order to prevent decoupling of first and second couplings 1750 and 1752 while a portion of elongate tube 1742 passes through the first and second lumens of first and second couplings 1750 and 1752, respectively.

For some applications, tube 1742 of decoupling-prevention element 1740 comprises a tube of an anchor-delivery system 1743. For some applications, anchor-delivery system 1743 comprises the deployment manipulator, anchor driver, and deployment element, as described in US Patent Application Publication 2014/0309661 to Sheps et al., and/or in US Patent Application Publication 2015/0272734 to Sheps et al., which are incorporated herein by reference.

For some applications, decoupling-prevention element 1740 comprises an overtube 1744, e.g., a steerable catheter, which is used to deliver annuloplasty structure 1722. Overtube 1744 has an inner diameter sized so as to maintain coupling of couplings 1750 and 1752 when a portion of overtube 1744 surrounds first and second couplings 1750 and 1752.

For some applications, decoupling-prevention element 1740 comprises contracting member 30. Since contracting member 30 extends along, e.g., threaded along, primary body portion 1723 and linking segments 1730, contracting member 30 maintains coupling of first and second couplings 1750 and 1752.

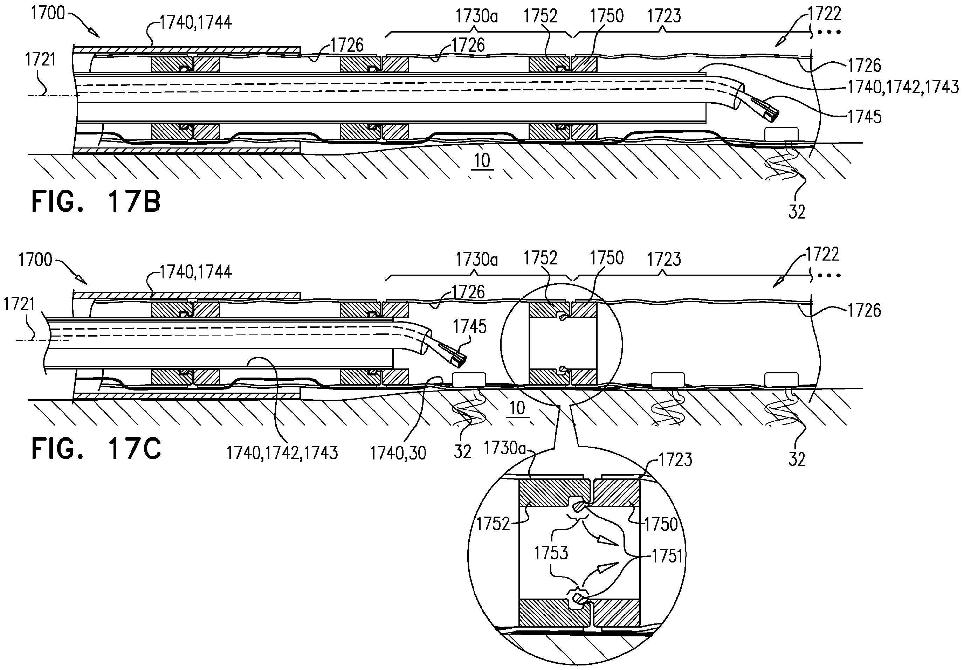

Fig. 17B shows anchor-delivery system 1743 delivering a tissue anchor 32 through a portion of sleeve 1726 of primary body portion 1723 and into cardiac tissue 10. System 1743 comprises an anchor driver 1745 which is reversibly coupled to anchor 32 and is decoupled therefrom following deployment of anchor 32.

Once primary body portion 1723 is anchored to cardiac tissue 10, anchor-delivery system 1743 is moved in order to position first linking segment 1730a appropriately at cardiac tissue 10 to be anchored thereto. As shown in Fig. 17C, a portion of tube 1742 of system 1743 is moved away from first and second couplings 1750 and 1752.

For applications in which one or more projections 1751 of first coupling 1750 is biased to move radially inward, in the absence of decoupling-prevention element 1740, projections 1751 move radially inward, as shown in Fig. 17C.

It is to be noted that for some applications, one or more projections 1751 of first coupling 1750 are not biased to move radially inward, and are only moveable in response to a force applied thereto, e.g., a pulling force applied to segment 1730a from a proximal direction. In such applications, one or more projections 1751 do not move radially inwardly in the absence of the pushing force applied thereto by the external surface of tube 1742.

In either application, contracting member 30 maintains coupling of linking segment 1730a and primary body portion 1723 even though tube 1742 is not disposed within the respective elements of couplings 1750 and 1752 and, as a result, one or more projections 1751 are moveable from within negative space 1753. In such a manner, contracting member 30 functions as decoupling-prevention element 1740. Additionally, since segment 1730a is anchored to tissue 10, coupling of segment 1730 and primary body portion 1723 is maintained.

In Fig. 17D, second linking segment 1730b is anchored to cardiac tissue 10 by anchor delivery system 1743, in a manner as described hereinabove with reference to Fig. 17C. Accordingly, since tube 1742 of decoupling-prevention element 1740 extends beyond the lumens of couplings 1750 and 1752 coupling first and second linking segments 1730a and 1730b, one or more projections 1751 of coupling 1750 of first linking segment 1730a is moveable from within negative space 1753 of coupling 1752 of second linking segment 1730b.

As noted hereinabove with reference to Fig. 17C, for applications in which one or more projections 1751 of first coupling 1750 is biased to move radially inward, in the absence of decoupling-prevention element 1740, projections 1751 move radially inward.

Additionally, as noted in Fig. 17C, it is to be noted that for some applications, one or more projections 1751 of first coupling 1750 are not biased to move radially inward, and are only moveable in response to a force applied thereto, e.g., a pulling force applied to segment 1730a from a proximal direction. In such applications, one or more projections 1751 do not move radially inwardly in the absence of the pushing force applied thereto by the external surface of tube 1742.

Fig. 17E shows decoupling of third linking segment 1730c from second linking segment 1730b.

As noted hereinabove with reference to Fig. 17C, for applications in which one or more projections 1751 of first coupling 1750 is biased to move radially inward, in the absence of decoupling-prevention element 1740, projections 1751 move radially inward.

Additionally, as noted in Fig. 17C, it is to be noted that for some applications, one or more projections 1751 of first coupling 1750 are not biased to move radially inward, and are only moveable in response to a force applied thereto, e.g., a pulling force applied to segment 1730c from a proximal direction. In such applications, one or more projections 1751 do not move radially inwardly in the absence of the pushing force applied thereto by the external surface of tube 1742.

In either application, third linking segments 1730c can be decoupled from second linking segment following radial movement of one or more projections 1751 of coupling 1750 of second linking segment 1730b from within negative space 1753 of coupling 1752 of third linking segment 1730c.

As shown, third linking segment 1730c, as well as any other additional linking segments 1730 disposed proximally to third linking segment 1730c, are moved proximally away from primary body portion 1723 and first and second linking segments 1730a and 1730b coupled to cardiac tissue. As shown, third linking segment 1730c, as well as any other additional linking segments 1730 disposed proximally to third linking segment 1730c are pulled along contracting member 30.

Fig. 17E shows structure 1722 following contracting thereof. After third linking segment 1730c, as well as any other additional linking segments 1730 disposed proximally to third linking segment 1730c are removed from the body of the subject, excess portions of contracting member 30 are cut and removed from the body of the patient. A bead or lock 1760 is coupled to contracting member 30.

Once the physician has positioned structure 1722 along the annulus and has removed any number of linking segments 1730, contracting member 30 can be used to contract annuloplasty structure 1722, for example, by actuating contracting mechanism 1040, which comprises and/or is coupled to contracting member 30 as described in US Patent Application Publication 2014/0309661 to Sheps et al., and/or in US Patent Application Publication 2015/0272734 to Sheps et al., which are incorporated herein by reference. Additionally, implantation of structure 1722 can be done using any methods described in the '661 and/or '734 applications.

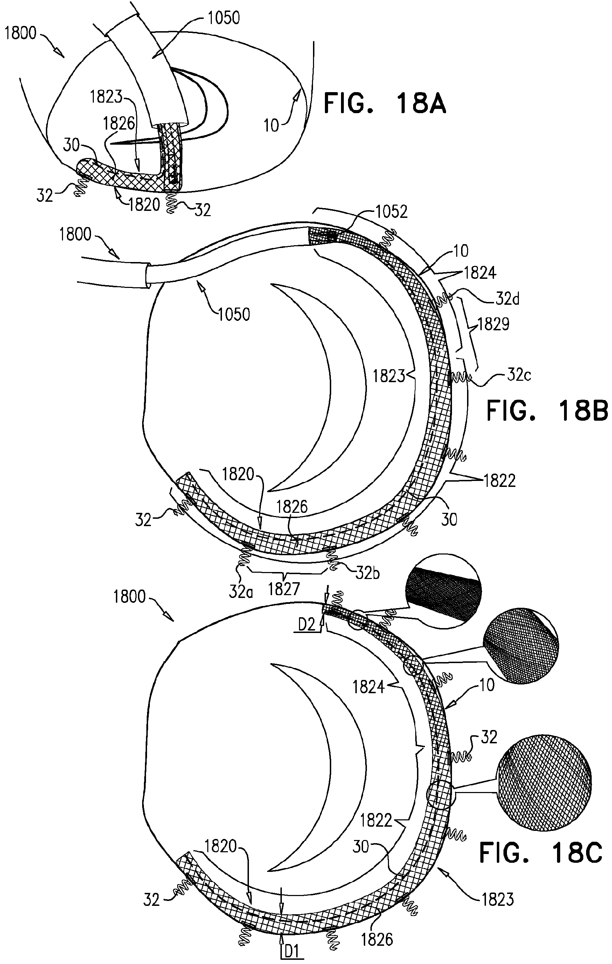

Reference is now made to Figs. 18A-C, which are schematic illustrations of an exemplary system 1800 comprising a variably-stretchable structure 1820 (e.g., an annuloplasty structure, an annuloplasty ring structure, etc.) having a flexible body portion 1823 (e.g., an annuloplasty body portion, an annuloplasty ring body portion, etc.) comprising a sleeve 1826 and a contracting member 30, in accordance with some applications. Annuloplasty structure 1820 can comprise any annuloplasty structure or annuloplasty ring structure described herein with reference to Figs. 1-17F. Additionally, for some applications, structure 1820 can comprise an actuatable contracting mechanism comprising and/or coupled to contracting member 30 as described in US Patent Application Publication 2014/0309661 to Sheps et al., and/or in US Patent Application Publication 2015/0272734 to Sheps et al., which are incorporated herein by reference. Additionally, implantation of structure 1820 can be done using any methods described in the '661 and/or '734 applications.

Sleeve 1826 can comprise a braided fabric mesh, e.g., comprising polyethylene terephthalate, Dacron (TM), other materials, etc. Body portion 1823 (e.g., sleeve 1826) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus (e.g., a full or closed ring). In order to tighten the annulus, the system comprises an actuatable adjustment mechanism or contracting mechanism. The adjustment mechanism or contracting mechanism can be part of and/or coupled to one or more of the annuloplasty structure 1820, a flexible elongate contracting member 30 that extends along sleeve 1826, and/or another component. Elongate contracting member 30 can comprise a wire, a ribbon, a rope, or a band, which can comprise a flexible and/or superelastic material, e.g., nitinol, polyester, stainless steel, or cobalt chromium. For some applications, the contracting member 30 comprises a radiopaque material. For some applications, contracting member 30 comprises a braided polyester suture (e.g., Ticron). For some applications, contracting member 30 is coated, such as with polytetrafluoroethylene (PTFE) or another polymer. For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

For some applications, sleeve 1826 is labeled with one or more radiopaque markers. Sleeve 1826 can comprise a flexible sleeve 1826 shaped and configured so as to define a lumen therethrough.

In Fig. 18A, an anchor-delivery system 1050 or other attachment system can be used to anchor or attach a first section of annuloplasty body portion 1823 of annuloplasty structure 1820, for example, by deploying one or more tissue anchors 32 through sleeve 1826 and into annulus tissue 10, from within a lumen of sleeve 1826. Techniques for deploying anchors 32 through sleeve 1826 can be practiced in combination with techniques described in WO 2013/069019 to Sheps, which is incorporated herein by reference. Other fastening or attachment means (e.g., staples, fasteners, clips, screws, adhesives, etc.) can also or alternatively be used.

In Fig. 18B, anchor-delivery system 1050 is used to deliver and anchor or attach additional sections of body portion 1823 of annuloplasty structure 1820. As shown, body portion 1823 comprises (1) a first segment 1822 having a first degree of stretchability, and (2) a second segment 1824 having a second degree of stretchability that is greater than the first degree of stretchability. For some applications, system 1050 can reversibly engage and reversibly grip to a portion of sleeve 1826 during the stretching of second segment 1824, e.g., in order to facilitate pulling of sleeve 1826.

A respective length of each of first and second segments 1822 and 1824 can be controlled by the operating physician.

For some applications, the entire body portion 1823 has a gradually increasing flexibility from a distal end of body portion 1823 toward a proximal end of body portion 1823. That is, second segment 1824 can be longer and/or assume a greater percentage of body portion 1823 than as shown in Figs. 18B-C by way of illustration and not limitation.

In the context of the present application, including the claims "proximal" refers to a part of the structure that is closer to the orifice through which the structure was introduced and "distal" refers to a part of the structure that is farther from the orifice through which the structure was introduced.

Additionally, for some applications, the increased stretchability of segment 1824 imparts increased compressibility to segment 1824. For example, body portion 1823 can be delivered to the heart in an elongate state, anchoring in part to the annulus, and then subsequently to the anchoring, shortening of the elongate structure (e.g., by compressing) in real time during the implantation procedure while maintaining the body portion of the structure or annuloplasty ring structure intact (e.g., by not cutting or severing any portion of the body portion), and subsequently to the shortening, contracting of the structure or annuloplasty ring structure by contracting member 30 in order to contract and remodel the annulus of the patient. For example, for some applications, after having fixed first segment 1822 to a first length of tissue of the annulus, it may become apparent to the physician that the length of path of the remaining tissue of the annulus is shorter than initially anticipated. With structure 1820 having segment 1824 with increased compressibility, the physician can the compress the remaining sections of second segment 1824 in order to accommodate the length of the path of the remaining tissue of the annulus.

Once the physician has fixed (e.g., typically by anchoring with anchor 32 and/or fastening with some other attachment means) first segment 1822 to the annulus, the physician subsequently stretches second segment 1824 in order to accommodate the remaining portions of the annulus to which structure 1820 has not yet been anchored. For example, for some applications, after having fixed first segment 1822 to a first length of tissue of the annulus, it may become apparent to the physician that the length of path of the remaining tissue of the annulus is longer than initially anticipated. With structure 1820 having segment 1824 with increased stretchability, the physician can the stretch second segment 1824 in order to accommodate the length of the path of the remaining tissue of the annulus. Subsequently to the stretching of second segment 1824, second segment 1824 is fixed to the annulus (e.g., one or more anchors 32 are deployed through sleeve 1826 of second segment 1824 in order to fix second segment 1824 to the annulus). In such a manner, a shorter annuloplasty structure can be delivered to the annulus and only stretched to a longer length once initially anchored to the annulus. As such, structure 1820 comprises a partially-stretchable, or partially-elastic structure 1820. For some applications, as body segment 1824 of body portion 1823 is stretched, a diameter of segment 1824 incrementally and gradually decreases.

As shown in Figs. 18B-C, first segment 1822 is designated for implantation between the vicinity of the anterolateral commissure and roughly P2 (by way of illustration and not limitation), while second segment 1824 is designated for implantation between roughly P2 (by way of illustration and not limitation) and the posterolateral commissure. Fig. 18C shows the various diameters of sleeve 1826. It is to be noted that although only second segment 1824 is shown as being more stretchable than first segment 1822, applications include an annuloplasty structure having a body portion that is has alternating segments of variable stretchability. Segment 1824 can be designated for implantation anywhere along the annulus.

Since first segment 1822 has a degree of stretchability that is lower than second segment 1824, first segment 1822 typically but not necessarily has a D1 greater than a diameter D2 of second segment 1824 following stretching, and typically before contraction of structure 1820.

For some applications, the diameter of segment 1824 does not decrease following stretching. In such applications, the diameter of segment 1824 remains the same or similar to the diameter of segment 1822. For some applications, only the inner diameter may be reduced in response to the stretching of segment 1824. For some applications, only the outer diameter may be reduced in response to the stretching of segment 1824. For either of these applications, appropriate materials can be used which help to control the diameter of a given section of structure 1820 responsively to the application of a pulling, stretching, or compressing force. These appropriate materials also help to control a thickness of the wall of sleeve 1826.

Typically, second segment 1824 is, overall, more stretchable than first segment 1822. For some applications, second segment 1824 is homogenous throughout. That means, that the entire segment 1824 is stretchable in equal measure along the entire length of segment 1824. For some applications, second segment 1824 can have sections that are more stretchable and sections which are less stretchable. For such applications, second segment 1824 can have increasing stretchability along its length, or, second segment 1824 can have sections of variable stretchability which alternate along the length of second segment 1824.

As shown by way of illustration and not limitation in Figs. 18B-C, second segment 1824 assumes roughly one-third of body portion 1824. It is to be noted that segments 1822 and 1824 can assume any proportion. For example, section 1824 can assume roughly 50% or 75% of body portion 1823. For some applications, body portion 1823 can be increasingly stretchable along the entire length of body portion 1823.

Although Figs. 18A-C shows sleeve 1826 comprising a braided fabric, sleeve 1826 can comprise other materials such as a flexible, tubular plastic, for example, that is variably-stretchable, or variably-elastic. For some applications, the sleeve can comprise a spiral design, for example, the sleeve can comprise flexible, tubular plastic that has a spiral design. For some applications, the spiral can have a variable pitch and/or diameter to help vary the flexibility of the sleeve in different regions.

Reference is now made to Figs. 18A-C. For first segment 1822, (1) anchors 32 (and/or another attachment means) can be deployed at an equal or similar distance from each other (e.g., a distance of 4-10 mm between each anchor/attachment means), and (2) an equal or similar length of sleeve 1826 can be disposed between each anchor/attachment means. For second segment 1824, (1) anchors 32 (or other attachment means) can be deployed at an equal or similar distance from each other, and (2) because sleeve 1826 is stretched, a smaller length of sleeve 1826 can be used to span the distance between each anchor 32 in second segment 1824 than the length of sleeve 1826 used to span the distance between anchors 32 in first segment 1822.

That is, for a method in which structure 1820 is implanted, first segment 1822 can be implanted by driving an anchor 32 through sleeve 1826 as shown in Fig. 18A (or using another attachment means). A first section of sleeve 1826 of first segment 1822 can be released from anchor delivery system 1050. The first section can then be positioned along tissue 10 and another anchor 32 can be driven through sleeve 1826 using an anchor driver 1052 in order to fix the first section of sleeve 1826 to tissue 10 (or this section can be fixed/attached to the tissue using another attachment means). Another section 1827 of sleeve 1826 of first segment 1822 can then be released from anchor delivery system 1050 and positioned along tissue 10 and another anchor 32a can be driven through sleeve 1826 (as is shown in Fig. 18B) (or the additional section 1827 can be fixed/attached to the tissue using another attachment means). Once the physician implants first segment 1822, the physician can expose a section 1829 of second segment 1824 by releasing section 1829 from delivery system 1050. Section 1829 of segment 1824 released from system 1050 has a length that is smaller than the length of section 1827 of segment 1822 released from system 1050. The physician can then stretch section 1829 to a stretched state having a length that is greater than the pre-stretched length of section 1829 released from delivery system 1050. The physician can position section 1829 in the stretched state along tissue 10 and can fix section 1829 of second segment 1824 to tissue 10, e.g., by an attachment means such as driving a tissue anchor 32d at a distance from the preceding anchor 32c that is equal or generally similar to the distance between anchors 32a and 32b used to anchor section 1827 of first segment 1822.

Since second segment 1824 has increased flexibility, (1) a smaller length of sleeve 1826 can be released from delivery system 1050 incrementally in order to span the distance between attachment means/anchors 32 used to fix second segment 1824, then (2) a length of sleeve 1824 can be released from delivery system 1050 in order to span the same distance between attachment means/anchors 32 of first segment 1822.

That is, if the physician stretches sleeve 1826 at section 1829 of second segment 1824 to the span same distance between each attachment means/anchor 32, a smaller length of sleeve 1826 is released from delivery system 1050 prior to stretching between each anchor 32 in second segment 1824 than the length of sleeve 1826 is released from delivery system 1050 in order to span the distance between anchors 32 in first segment 1822.

For some applications, the stretching of segment 1824 straightens sleeve 1826 prior to anchoring of sleeve 1826. This straightening of sleeve 1826 by stretching advantageously lowers the likelihood of folding of sleeve 1826 in the anchoring region, thereby minimizing interference of any folds of sleeve 1826 with anchors 32.

It is to be further noted, that even though sleeve 1826 is stretched at second segment 1824, the elastic force of second segment 1824 (i.e., the force exerted by sleeve 1826 in order to return to its original length following anchoring of segment 1824) can be selected and/or configured to be insufficiently strong to contract the annulus. Thereby, annuloplasty is only achieved when sleeve 1826 is intentionally contracted by the physician, e.g., using contracting member 30. That is, the elastic force of second segment 1824 is not strong enough to contract second segment 1824 enough that it will affect tissue 10 to which second segment 1824 is coupled.

Reference is again made to Figs. 18A-C. System 1800 minimizes the likelihood of any excess sleeve 1826 interfering with the cardiac valve. As shown in Fig. 18B, once first segment 1822 has been anchored, second segment 1824 is expanded by being stretched, and subsequently, anchors 32 are used to anchor second segment 1824 to the cardiac tissue. Additionally, with system 1800, the physician does not need to measure the size of the annulus prior to positioning annuloplasty structure 1820. In such a manner, structure 1820 accommodates and fits all native annulus sizes.

Once the physician has positioned structure 1820 along the annulus and has expanded segment 1824, contracting member 30 is used to contract annuloplasty structure 1820. For some applications, contracting member 30 is coupled to an actuatable contracting mechanism as described in US Patent Application Publication 2014/0309661 to Sheps et al., and/or in US Patent Application Publication 2015/0272734 to Sheps et al., which are incorporated herein by reference. Additionally, implantation of structure 1820 can be done using any methods described in the '661 and/or '734 applications.

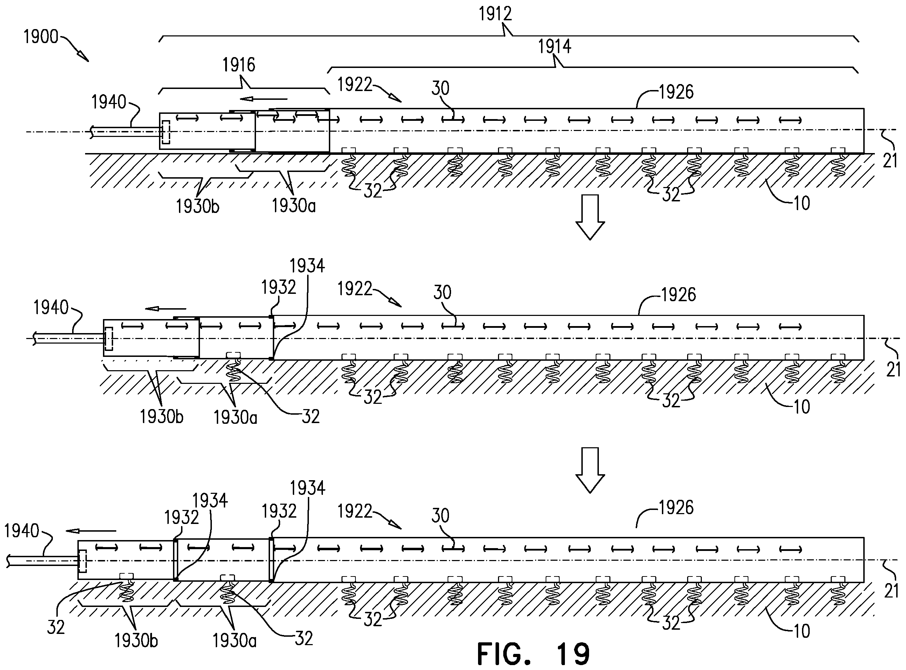

Fig. 19 is a schematic illustration of an exemplary system 1900 comprising a structure 1922 (e.g., an annuloplasty structure, an annuloplasty ring structure, etc.) having a body portion 1912 (e.g., an annuloplasty body portion, an annuloplasty ring body portion, etc.) comprising a sleeve 1926 and a contracting member 30, in accordance with some applications. For some applications, sleeve 1926 is tapered. Sleeve 1926 is shaped and configured so as to define a lumen therethrough. For some applications, body portion 1912 is flexible.

Structure 1922 comprises one or more (e.g., two as shown by way of illustration and not limitation) telescoping segments 1930a and 1930b. It is to be noted that structure 1922 can comprise any number of telescoping segments 1930 having any length as determined by a physician. For example, for some applications, all segments 1930 can have the same length. For other applications, segments 1930 can have a variable length. Structure 1922 can be delivered to the heart in a configuration in which telescoping segment 1930b is disposed at least in part within a lumen of telescoping segment 1930a, and telescoping segment 1930a is disposed at least in part within the lumen of sleeve 1926. Telescoping segments 1930a and 1930b can be moveable, or slidable, along a longitudinal axis 21 of body portion 1912.

A proximal end of sleeve 1926 comprises a coupling element 1934 which is configured to engage and become coupled to a coupling element 1932 at a distal end of telescoping segment 1930a. A proximal end of telescoping segment 1930a comprises a coupling element 1934 which is configured to engage and become coupled to a coupling element 1932 at a distal end of telescoping segment 1930b. For some applications, coupling elements 1932 and 1934 comprise hook-and-loop fasteners. For some applications, coupling elements 1932 and 1934 comprise magnets. Coupling elements 1932 and 1934 can comprise ring-shaped coupling elements which provide an unobstructed passage through the lumen of the entire structure 1922. Coupling elements 1932 and 1934 are coupled together typically following movement of the telescoping segment 1930 into its final position.

For some applications, telescoping segments 1930 comprise the same material as sleeve 1926. For some applications telescoping segments 1930 comprise material that is different from the material of sleeve 1826. For some applications, telescoping segments 1930 can have a wall thickness that is lower than the wall thickness of sleeve 1826.

Annuloplasty structure 1922 can comprise any annuloplasty structure or annuloplasty ring structure described herein with reference to Figs. 1-18C. Additionally, for some applications, structure 1922 can comprise an actuatable contracting mechanism comprising and/or coupled to contracting member 30 as described in US Patent Application Publication 2014/0309661 to Sheps et al., and/or in US Patent Application Publication 2015/0272734 to Sheps et al., which are incorporated herein by reference.

Sleeve 1926 and telescoping segments 1930 can comprise a braided fabric mesh, e.g., comprising polyethylene terephthalate, Dacron (TM), other materials, etc. Sleeve 1926 and telescoping segments 1930 can be configured to be placed only partially around a cardiac valve annulus (i.e., to assume a C-shape), and, once anchored in place, to be contracted so as to circumferentially tighten the valve annulus. Optionally, the ring structure can be configured to be placed entirely around the valve annulus (e.g., a full or closed ring). In order to tighten the annulus, the system comprises an actuatable adjustment mechanism or contracting mechanism. The adjustment mechanism or contracting mechanism can be part of and/or coupled to one or more of the annuloplasty structure 1922, a flexible elongate contracting member 30 that extends along sleeve 1926 and telescoping segments 1930, and/or another component. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated, such as with polytetrafluoroethylene (PTFE) or another polymer. For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents incorporated by reference.

For some applications, sleeve 1926 and telescoping segments 1930 are labeled with one or more radiopaque markers. Typically, sleeve 1926 and telescoping segments 1930 comprises a flexible sleeve 1926 shaped and configured so as to define a lumen therethrough.

An anchor-delivery system 1940 or other attachment system can be used to anchor or attach a first segment 1914 of annuloplasty body portion 1912 of annuloplasty structure 1922, for example, by deploying one or more tissue anchors 32 through sleeve 1926 and into annulus tissue 10, from within a lumen of sleeve 1926. Techniques for deploying anchors 32 through sleeve 1926 can be practiced in combination with techniques described in WO 2013/069019 to Sheps, which is incorporated herein by reference.

As shown, body portion 1912 comprises (1) first segment 1912, and (2) a second segment 1916 comprising telescoping segments 1930. For some applications, anchor-delivery system 1940 can reversibly engage and reversibly grip to a portion of telescoping segments 1930 during the pulling and moving of telescoping segment 1930.

Any number of tissue anchors 32 or other fastening or attachment means can be used to anchor or attach structure 1922 to the annulus of the valve. In some embodiments, once a sufficient amount of first segment 1914 of body portion 1912 is anchored/attached to the annulus, the physician pulls and expands telescoping segment 1930a from within the lumen of sleeve 1926 using system 1940. Once telescoping segment 1930a has been pulled from within the lumen of sleeve 1926, it is anchored/attached to annulus tissue 10 using an attachment means/tissue anchor 32, as shown. The physician then pulls and expands telescoping segment 1930b from within the lumen of telescoping segment 1930a using system 1940. Once telescoping segment 1930b has been pulled from within the lumen of telescoping segment 1930a, it can be anchored/attached to annulus tissue 10 using an attachment means/tissue anchor 32, as shown. In such a manner, system 1900 minimizes the likelihood of any excess sleeve 1926 interfering with the cardiac valve. Additionally, with system 1900, the physician does not need to measure the size of the annulus prior to positioning annuloplasty ring structure 1922. In such a manner, structure 1922 accommodates and fits all native annulus sizes.

First segment 1914 can be designated for implantation between the vicinity of the anterolateral commissure and roughly P2, while second segment 1916 can be designated for implantation between roughly P2 and the posterolateral commissure. Telescoping segments 1930 can be located at any part of body portion 1912.

Once the physician has positioned structure 1922 along the annulus and has secured telescoping segments 1930, contracting member 30 is used to contract annuloplasty structure 1922. For some applications, contracting member 30 is coupled to an actuatable contracting mechanism as described in US Patent Application Publication 2014/0309661 to Sheps et al., and/or in US Patent Application Publication 2015/0272734 to Sheps et al., which are incorporated herein by reference. Additionally, implantation of structure 1820 can be done using any methods described in the '661 and/or '734 applications.

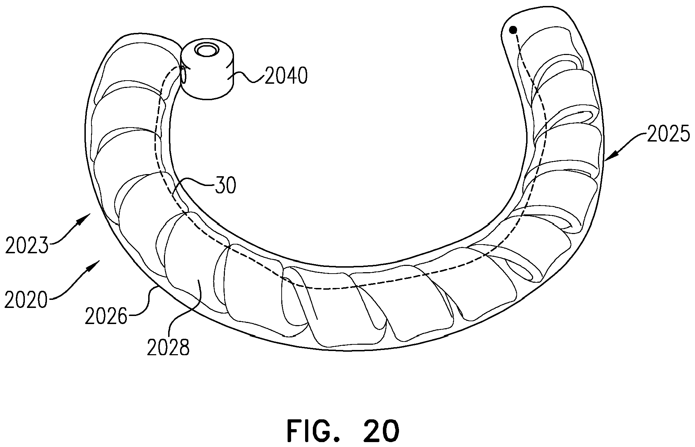

Reference is made to Figs. 20, 21A-B, 22A-C, and 23, which are schematic illustrations of an annuloplasty system 2000, and use thereof, in accordance with some applications. System 2000 comprises an elongate annuloplasty structure 2020 having a flexible body portion 2023 (e.g., an annuloplasty body portion, an annuloplasty ring body portion, etc.). Annuloplasty structure 2020 can comprise any annuloplasty structure or annuloplasty ring structure described herein. Typically, annuloplasty structure 2020 comprises contracting member 30 for contracting body portion 2023 after implantation of the annuloplasty structure. Contracting member 30 extends along body portion 2023. Additionally, for some applications, the system can comprise an actuatable adjustment mechanism 2040. The adjustment mechanism can be part of and/or coupled to one or more of the structure 2020, contracting member 30, and/or another component, e.g., as described elsewhere herein and/or as described in US Patent Application Publication 2014/0309661 to Sheps et al., and/or in US Patent Application Publication 2015/0272734 to Sheps et al., which are incorporated herein by reference. Optionally, structure 2020 (and the other annuloplasty structures described herein) can be contracted by tensioning contracting member 30 by pulling on the contraction member, and using a locking element, such as a crimp, to lock in the tension. Implantation of structure 2020 can be performed using any methods described in the '661 and/or '734 applications.

Annuloplasty structure 2020 (e.g., body portion 2023) comprises a tubular wall 2025 that defines a lumen along body portion 2023. That is, the lumen is defined along a longitudinal axis of elongate annuloplasty structure 2020. Wall 2025 comprises a sleeve 2026 and a helical member 2028. Sleeve 2026 can comprise a braided fabric mesh, e.g., comprising polyethylene terephthalate, Dacron (TM), other materials, etc. Body portion 2023 (e.g., wall 2025, such as sleeve 2026) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus (e.g., a full or closed ring). For some applications, body portion 2023 (e.g., sleeve 2026) is labeled with one or more radiopaque markers, e.g., as described hereinabove, *mutatis mutandis.*

Helical member 2028 defines a plurality of helical turns extending in a helical path along and around the lumen of body portion 2023. Helical member 2028 is coupled to sleeve 2026. For example, helical member 2028 (e.g., parts thereof, or the entirety thereof) can be stitched or glued to sleeve 2026. Structure 2020 is shown with helical member 2028 being disposed inside of sleeve 2026, but the helical member can optionally be disposed outside of the sleeve. For some applications, the helical member can be embedded within the sleeve.

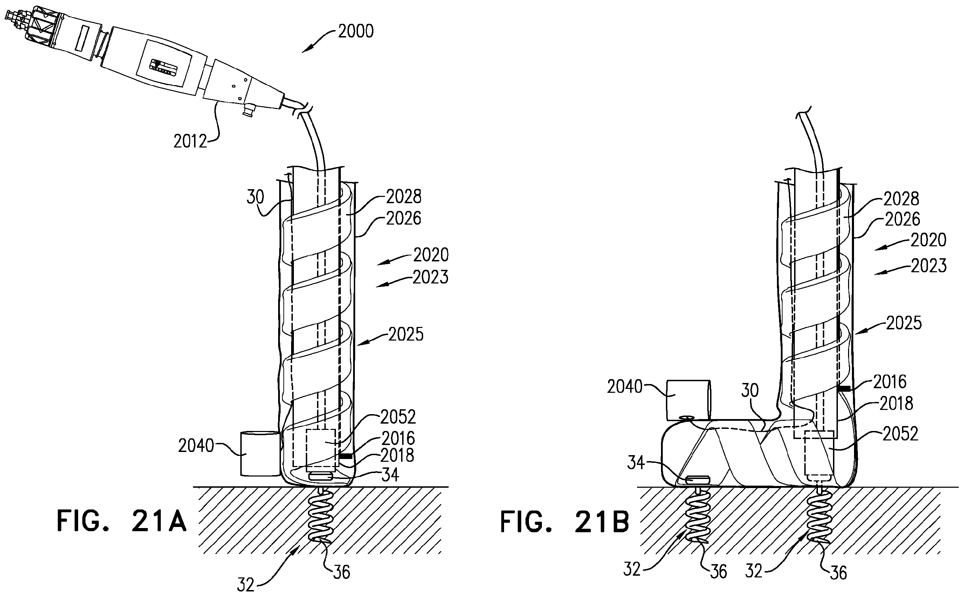

Many other annuloplasty structures described herein are described as being anchorable to tissue by anchors 32 being driven through the sleeve and into the tissue. Therefore, in some applications, such sleeves are configured to be sufficiently strong to support the resulting forces at the point of anchoring (e.g., at the point at which the anchor pierces the sleeve). As described hereinabove (e.g., with reference to Fig. 10), there is typically a trade-off between (i) strength, rigidity, and/or stability of a sleeve, and (ii) compressibility and/or stretchability of that sleeve. In order to provide increased longitudinal compressibility of body portion 2023 (e.g., so as to be adaptable to various annulus sizes), sleeve 2026 is made from a material (e.g., a fabric) that is particularly flexible, compressible, and/or stretchable (e.g., elastically stretchable). As a result, this material can be thinner, less dense, and/or weaker than that of other sleeves described herein. To accommodate this, body portion 2023 comprises helical member 2028, which is stronger, and typically stiffer than sleeve 2026. Annuloplasty structure 2020 is anchored to tissue by anchors 32 holding helical member 2028 to the tissue, typically by being driven through the helical member (Figs. 21A-B). Helical member 2028 thereby provides the strength required for anchoring annuloplasty structure 2020 and performing annuloplasty by contracting the annuloplasty structure, while sleeve 2026 continues to serve other functions that are served by other sleeves described herein, such as providing a closed lumen that reduces a likelihood of an anchor becoming an embolus.

For some applications, helical member 2028 comprises a polymer. For some applications, helical member 2028 is metallic. For some applications, helical member 2028 comprises a shape memory material. For some applications, helical member 2028 comprises a fabric.

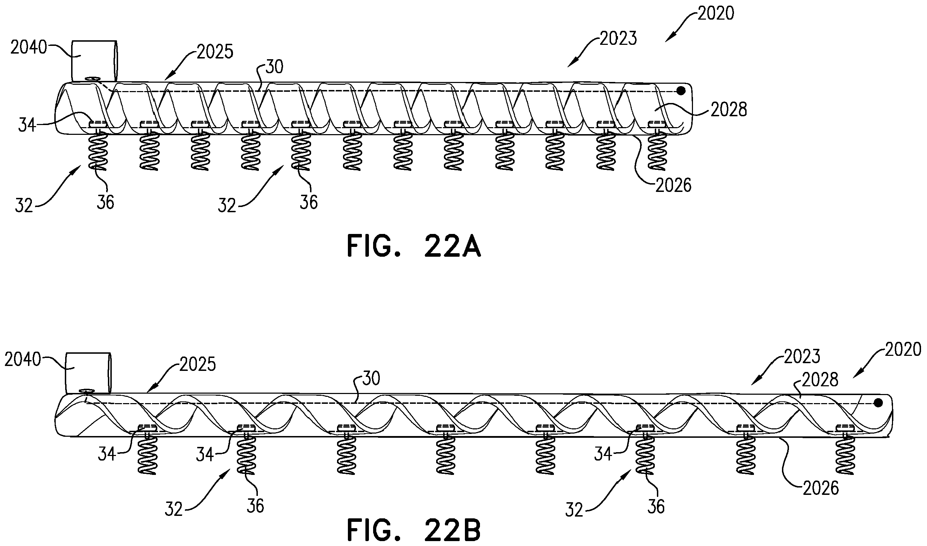

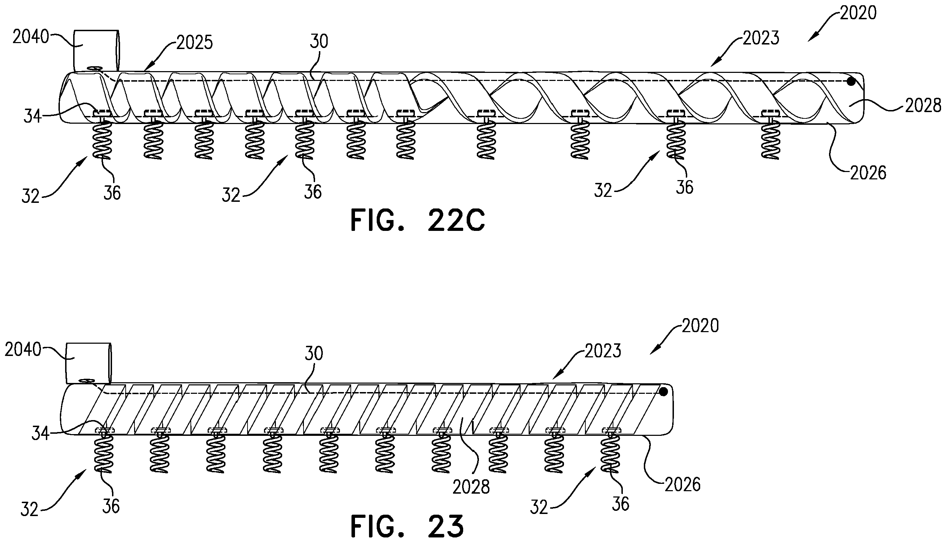

Helical member 2028 allows annuloplasty structure 2020 to be implanted relatively compressed for a relatively small annulus, with the helical member having a relatively small helix pitch (Fig. 22A), or relatively stretched for a relatively large annulus, with the helical member having a relatively small helix pitch (Fig. 22B). Furthermore, if during implantation, it is identified that the annulus is larger or smaller than originally understood, the operating physician can dynamically adapt the degree of compression/stretching for subsequently anchored parts of body portion 2023. Fig. 22C represents one example of such dynamic adaptation, in which, after seven anchors 38 had been anchored, it was identified that the annulus was larger than originally anticipated, and the operating physician dynamically increased the degree of stretching for the subsequent anchors.

For some other annuloplasty structures described herein, contracting member 30 extends along the body portion of the annuloplasty structure by weaving along the sleeve of the annuloplasty structure. In contrast, due to particular nature of sleeve 2026, for annuloplasty structure 2020 contracting member 30 extends through the lumen of body portion 2023, i.e., radially inward from helical member 2028.

Figs. 21A-B show steps in the implantation of annuloplasty structure 2020, in accordance with some applications. Attachment means, such as a plurality of anchors 38 can be used. In some implementations, each anchor 38 comprises a head 34 and a tissue-engaging element 36. Tissue-engaging element 36 is shown as a helical tissue-engaging element that is screwed into tissue, but can be any suitable type of tissue-engaging element, including a dart or a staple. Each anchor 32 is dimensioned to be advanceable along (i.e., longitudinally through) the lumen of body portion 2023, typically with tissue-engaging element 36 disposed distally to head 34. An anchor driver 2052 is reversibly couplable to anchor 32 (e.g., to head 34 thereof), and is configured to advance the anchor along the lumen (typically introducing the anchor into the lumen via an open proximal end of the lumen). In some implementations, anchor driver 2052 uses each anchor to secure a respective helical turn of helical member 2028 to the tissue by driving tissue-engaging element 36, from the lumen, through wall 2025, and into the tissue, such that the helical turn becomes sandwiched between head 34 and the tissue. As shown in Figs. 21A-B, tissue-engaging element 36 can be driven through helical member 2028 itself.

For some applications, and as shown in Fig. 23, tissue-engaging element 36 or at least some (or all) of the tissue-engaging elements can be driven through wall 2025 adjacent to the helical turn to be anchored (e.g., between helical turns), but sufficiently close to a helical turn that the helical turn becomes sandwiched between head 34 and the tissue. Fig. 23 shows adjacent helical turns being anchored by a single anchor 32 disposed between them. For some such applications, head 34 is wider than for applications in which tissue-engaging element 36 is driven through the helical member itself.

For some applications, every helical turn of helical member 2028 is secured to the tissue by a respective anchor 32 (e.g., as shown in Figs. 22A-C). For some applications, not every helical turn is secured by a respective anchor. For example, Fig. 21B shows a non-anchored helical turn disposed between two anchored helical turns.

Subsequent to its anchoring to the annulus, annuloplasty structure 2020 is contracted to reduce the size of the annulus, e.g., as described hereinabove, *mutatis mutandis.* Helical member 2028 and sleeve 2026 also facilitate this, with the helix pitch of the helical member becoming smaller during contraction.

For some applications, and as shown, system 2000 comprises a tool 2012 that facilitates delivery and/or anchoring of annuloplasty structure 2020. For some applications, anchor driver 2052 is a component of tool 2012. For some applications, tool 2012 comprises an anchor channel 2018 that extends into (and often through) the lumen of body portion 2023. For such applications, annuloplasty structure 2020 is typically delivered with channel 2018 already in place. Each anchor 32 is advanced into and through the lumen of body portion 2023 by being advanced through channel 2018. The anchor is typically anchored by advancing the anchor out of the distal opening of the channel and through the part of wall 2025 that is directly in front of the distal opening. After each part of body portion 2023 is anchored, a subsequent part of the body portion is advanced off of channel 2018 (e.g., by retracting channel 2018 with respect to the body portion), and that subsequent part is then anchored.

Control of this advancement can be facilitated by cooperation between channel 2018 and helical portion 2023. For example, when moving channel 2018 to the next tissue site to which annuloplasty structure 2020 is to be anchored, the resulting tension on body portion 2023 can tighten helical member 2028 around the channel, thereby gripping the channel, and increasing control over the advancement of the body portion off of the channel. For some applications, tool 2012 comprises a protrusion 2016 that extends radially outward from channel 2018, and engages helical member 2028, inhibiting the helical turn with which it engages from sliding off of the channel (Figs. 21A-B). Advancement of body portion 2023 off of channel 2018 can thereby be controlled by revolution of protrusion 2016 around a central longitudinal axis of the channel. For applications in which protrusion 2016 is rigidly fixed to channel 2018, this revolution is achieved by rotating channel 2018 about its longitudinal axis. Typically, one full revolution of protrusion 2016 (e.g., by one full rotation of channel 2018), optionally in combination with proximal retraction of channel 2018, allows one full turn of helical member 2028 (and the associated part of body portion 2023) to be advanced off of channel 2018.

For some applications, protrusion 2016 is movable with respect to channel 2018, thereby providing a further degree of adjustability and/or control over the advancement of body portion 2023 off of the channel. For example, and as shown in Figs. 21A-21B, protrusion 2016 can be longitudinally slidable with respect to channel 2018. Alternatively or additionally, protrusion 2016 can be revolvable circumferentially around channel 2018 independently of rotation of the channel itself.

Reference is now made to Figs. 1-23. Components of any system and/or apparatus shown or described herein can be combined with any other apparatuses or embodiments shown or described herein. For example, the fabric shown in Fig. 9 can be used in combination with structure 522 shown in Figs. 8A-B. Steps from the various methods shown or described herein can also be combined and arranged in a variety of ways.

Reference is again made to Figs. 1-23. Systems 20, 120, 220, 250, 320, 420, 620, 720, 820, 920, 1000, 1600, 1700, 1800, 1900, and 2000, and methods 1200 and 1300 for repairing a dilated annulus of the patient can be used to treat any cardiac valve of the patient, e.g., the aortic valve, the pulmonary valve, the mitral valve, and the tricuspid valve. Further, the methods, operations, steps, etc. described herein can be performed on a living animal or on a non-living cadaver, cadaver heart, simulator, anthropomorphic ghost, etc. Systems described herein for treatment of valves can be used to treat other annular muscles within the body of the patient. For example, the systems described herein can be used in order to treat a sphincter muscle within a stomach of the patient.

Reference is again made to Figs. 1-23. It is to be further noted that systems 20, 120, 220, 250, 320, 420, 620, 720, 820, 920, 1000, 1600, 1700, 1800, 1900, and 2000 can be anchored or attached to tissue (e.g., of the annulus) using any of the anchoring devices and/or attachment means described in US Patent Application Publication 2015/0272734 to Sheps et al., including the anchor driver and the deployment manipulator, or otherwise described herein.

Reference is again made to Figs. 1-23. The systems 20, 120, 220, 250, 320, 420, 620, 720, 820, 920, 1000, 1600, 1700, 1800, 1900, and 2000, and methods 1200 and 1300 (or subcomponents thereof) and methods described hereinabove can also be used on any suitable tissue of the patient (e.g., stomach tissue, urinary tract, and prostate tissue).

Additionally, the scope herein includes applications described in one or more of the following:
- US Patent Application 12/435,291 to Maisano et al., entitled, "Adjustable repair chords and spool mechanism therefor," filed on May 4, 2009, which issued as US Patent 8,147,542;
- US Patent Application 12/437,103 to Zipory et al., entitled, "Annuloplasty ring with intra-ring anchoring," filed on May 7, 2009, which issued as US Patent 8,715,342;
- US Patent Application 12/548,991 to Maisano et al., entitled, "Implantation of repair chords in the heart," filed on August 27, 2009, which issued as US Patent 8,808,368;
- PCT Patent Application PCT/IL2009/001209 to Cabin et al., entitled, "Adjustable annuloplasty devices and mechanisms therefor," filed on December 22, 2009, which published as PCT Publication WO 10/073246;
- PCT Patent Application PCT/IL2010/000357 to Maisano et al., entitled, "Implantation of repair chords in the heart," filed on May 4, 2010, which published as WO 10/128502;
- PCT Patent Application PCT/EL2010/000358 to Zipory et al., entitled, "Deployment techniques for annuloplasty ring and over-wire rotation tool," filed on May 4, 2010, which published as WO 10/128503;
- US Patent Application Publication 2014/0309661 to Sheps et al.; and/or
- US Patent Application Publication 2015/0272734 to Sheps et al.

All of these applications are incorporated herein by reference. Techniques described herein can be practiced in combination with techniques described in one or more of these applications.

It will be appreciated by persons skilled in the art that the present invention is not limited to what has been particularly shown and described hereinabove. Rather, the scope of the present invention includes both combinations and subcombinations of the various features and steps described hereinabove, as well as variations and modifications thereof that are not in the prior art, which would occur to persons skilled in the art upon reading the foregoing description.

The invention further comprises the following embodiments:
1. An apparatus, comprising:
   an annuloplasty structure comprising:
   a body portion comprising flexible material, the body portion being shaped to define one or more controllably-expandable sections, each one of the one or more controllably-expandable sections having a respective first length;
   one or more control wires coupled to the one or more controllably-expandable sections, the one or more control wires being movable to facilitate expansion of each one of the one or more controllably-expandable sections to assume a respective second length that is greater than the respective first length; and
   a contracting member coupled to the body portion, the contracting member being configured to contract the body portion independently of the one or more control wires.
2. The apparatus according to embodiment 1, wherein each one of the one or more controllably-expandable sections comprises a part of the flexible material of the annuloplasty ring body portion, the part being folded, and wherein the one or more control wires is threaded through folds of the part.
3. The apparatus according to embodiment 2, wherein the part is folded telescopically in a direction away from a longitudinal axis of the body portion at the part.
4. The apparatus according to embodiment 2, wherein the part is folded in a manner in which at least one pouch is formed between layers of folds of the part, and wherein the pouch has a longitudinal length that is measured along a pouch axis that is generally parallel with a longitudinal axis of the body portion at the part.
5. The apparatus according to any one of embodiments 1 to 4, wherein the one or more control wires is removably coupled to the one or more controllably-expandable sections, and wherein the flexible material is biased such that the one or more controllably-expandable sections expand upon decoupling of the one or more control wires from the one or more controllably-expandable sections.
6. The apparatus according to any one of embodiments 1 to 4, wherein the one or more control wires is fixedly attached to the one or more controllably-expandable sections, and wherein the one or more control wires facilitates expansion of the one or more controllably-expandable sections responsively to pulling on the one or more control wires.
7. The apparatus according to any one of embodiments 1 to 6, wherein the one or more controllably-expandable sections comprise a plurality of controllably-expandable sections.
8. The apparatus according to embodiment 7, wherein the one or more control wires comprises exactly one control wire which controls all of the plurality of controllably-expandable sections.
9. The apparatus according to embodiment 7, wherein the one or more control wires comprises a plurality of control wires, each one of the plurality of control wires being movable to facilitate expansion of a respective one of the plurality of controllably-expandable sections.
10. Apparatus, comprising:
   an annuloplasty structure comprising:
   a body portion being shaped to define:
   a plurality of compressible sections, each one of the plurality of compressible sections having a first thickness; and
   a plurality anchor-designated sections, each one of the plurality of anchor-designated sections having a second thickness that is greater than the first thickness of a respective one of the plurality of compressible sections, the plurality anchor-designated sections being disposed alternatingly with the plurality of compressible sections, each anchor-designated section being designated for delivery of a respective tissue anchor therethrough; and
   a contracting member coupled to the body portion, the contracting member being configured to contract the body portion, the plurality of compressible sections facilitating a reduction in overall compressive forces on the body portion during contracting of the body portion by the contracting member.
11. The apparatus according to embodiment 10, wherein the first thickness is 0.05-0.1 mm and the second thickness is 0.15-0.2 mm.
12. The apparatus according to embodiment 10, wherein each one of the plurality of compressible sections is shaped so as to define a window.
13. The apparatus according to any one of embodiments 10 to 12, wherein the body portion comprises at least one flexible material, the at least one flexible material defining the plurality of compressible sections and the plurality of anchor-designated sections.
14. The apparatus according to any one of embodiments 10 to 12, wherein the body portion comprises:
   at least one first flexible material defining the plurality of compressible sections; and
   at least one second flexible material defining the plurality of anchor-designated sections.
15. The apparatus according to any one of embodiments 10 to 12, wherein each one of the plurality of compressible sections is shaped so as to define a compressible-section wall, and wherein the compressible-section wall has the first thickness.
16. The apparatus according to any one of embodiments 10 to 15, wherein each one of the plurality of anchor-designated sections is shaped so as to define an anchor-designated wall, and wherein the anchor-designated wall has the second thickness.
17. Apparatus, comprising:
   an annuloplasty structure comprising:
   a body portion having first and second body portion ends and comprising flexible material, the flexible material of the body portion being shaped to define:
   an attachment region or anchor-coupling region between the first body portion end and a vicinity of the body portion between the first and second body portion ends, exclusive of the second body portion end; and
   a deformable region between the second body portion end and a vicinity of the body portion between the first and second body portion ends, exclusive of the first body portion end, the deformable region being deformable in an absence of force applied thereto, the deformable region being deformable in order to shorten an overall length of the body portion.
18. The apparatus according to embodiment 17, further comprising a contracting member coupled to the body portion, the contracting member being configured to contract at least the attachment region or anchor-coupling region of the body portion.
19. The apparatus according to any one of embodiments 17 to 18, wherein the deformable region is deformable into a spiral configuration in the absence of the force applied to the deformable region.
20. The apparatus according to any one of embodiments 17 to 19, wherein the deformable region comprises nitinol, and wherein the nitinol is configured to facilitate deforming of the deformable region.
21. The apparatus according to any one of embodiments 17 to 20, wherein the deformable region assumes a linear configuration when a tube is disposed through at least a lumen of the deformable region.
22. The apparatus according to embodiment 21, wherein the tube comprises an anchor-delivery system.
23. Apparatus, comprising:
   an annuloplasty structure comprising:
   a body portion comprising a first flexible material, the body portion being shaped to define:
      a plurality of windows in the flexible material; and
      a plurality anchor-designated sections of the flexible material, the plurality of anchor-designated sections being disposed alternatingly with the plurality of windows, each anchor-designated section being designated for delivery of a respective tissue anchor therethrough;
   a plurality of covers comprising a second flexible material, the each one of the plurality of covers covering a respective one of the plurality of windows; and
   a contracting member coupled to the body portion and to the plurality of covers, the contracting member being configured to contract the body portion, the plurality of windows in the flexible material facilitating a reduction in overall compressible forces on the flexible material of the body portion along the body portion during contracting of the body portion by the contracting member.
24. The apparatus according to embodiment 23, wherein each one of the plurality of windows is shaped so as to define a longest dimension of 12-16 mm.
25. The apparatus according to any one of embodiments 23 to 24, wherein the first flexible material has a first thickness, and wherein the second flexible member has a second thickness, the first thickness being greater than the second thickness.
26. The apparatus according to embodiment 25, wherein the first thickness is 0.05-0.1 mm and the second thickness is 0.15-0.2 mm.
27. The apparatus according to any one of embodiments 23 to 26, wherein the second flexible material comprises:
   a first portion of the plurality of interwoven fibers oriented at an angle of 20 - 70 degrees with respect to a longitudinal axis of the body portion; and
   a second portion of the plurality of interwoven fibers oriented at an angle of 110 - 160 degrees with respect to the longitudinal axis of the body portion.
   a third portion of the plurality of interwoven fibers oriented at an angle of 0 - 15 degrees with respect to the longitudinal axis of the body portion; and
   a fourth portion of the plurality of interwoven fibers oriented at an angle of 75 - 90 degrees with respect to the longitudinal axis of the body portion.
28. Apparatus, comprising:
   an annuloplasty structure having a longitudinal axis, the annuloplasty structure comprising:
      a primary body portion;
      one or more linking segments removably coupled to the primary body portion;
      a first coupling coupled to a first end of the primary body portion;
      a second coupling coupled to a first end of a first linking segment of the one or more linking segments that is disposed adjacent to the first coupling of the first end of the primary body portion, the first and second couplings being shaped so as to mate with each other; and
      a decoupling-prevention element extending longitudinally along at least the first and second couplings in order to prevent decoupling of the first and second couplings,
   wherein removal of the decoupling-prevention element from at least the first and second couplings facilitates decoupling of the first and second couplings.
29. The apparatus according to embodiment 28, wherein at least one of the primary body portion and the one or more linking segments is flexible.
30. The apparatus according to any one of embodiments 28 to 29, further comprising an elongate contracting member extending along a longitudinal length of the primary body portion and along the one or more linking segments.
31. The apparatus according to any one of embodiments 28 to 29, wherein the decoupling-prevention element comprises an elongate contracting member extending along a longitudinal length of the primary body portion and the one or more linking segments.
32. The apparatus according to any one of embodiments 28 to 31, further comprising a contracting mechanism, coupled to the annuloplasty structure and configured to contract at least a portion of the implant structure.
33. The apparatus according to any one of embodiments 28 to 32, wherein:
   the first coupling is shaped so as to define a first lumen and comprises one or more radially-moveable projections, the one or more radially-moveable projections being radially moveable in response to a force applied thereto, and
   the second coupling is shaped so as to define a second lumen and a negative space shaped so as to receive the one or more radially-moveable projections.
34. The apparatus according to any one of embodiments 28 to 32, wherein:
   the first coupling is shaped so as to define a first lumen and comprises one or more radially-moveable projections, the one or more radially-moveable projections being biased to collapse radially inwardly in an absence of force applied thereto;
   the second coupling is shaped so as to define a second lumen and a negative space shaped so as to receive the one or more radially-moveable projections; and
   the decoupling-prevention element comprises an elongate tube configured to maintain the one or more radially-moveable projections within the negative space of the second coupling in order to prevent decoupling of the first and second couplings while a portion of the elongate tube passes through the first and second lumens of the respective first and second couplings.
35. The apparatus according to embodiment 34, wherein the elongate tube comprises a portion of an anchor-delivery system, and wherein the anchor-delivery system is configured to deploy a tissue anchor through the one or more linking segments.
36. The apparatus according to embodiment 34, wherein:
   the one or more linking segments comprises at least the first linking segment and at least a second linking segment,
   the first linking segment comprises a third coupling coupled to a second of the first linking segment,
   the second linking segment comprises a fourth coupling coupled to a first end of the second linking segment that is disposed adjacent to the first linking segment, the fourth coupling being shaped so as to mate with the third coupling,
   the decoupling-prevention element extends longitudinally at least through the third and fourth couplings in order to prevent decoupling of the third and fourth couplings, and
   removal of the decoupling-prevention element from at least the third and fourth couplings facilitates decoupling of the third and fourth couplings.
37. Apparatus, comprising:
   an annuloplasty structure comprising:
   a body portion being shaped to define:
      a first segment having a first degree of stretchability; and
      a second segment having a second degree of stretchability that is greater than the first degree of stretchability; and
   a contracting member coupled to the body portion, the contracting member being configured to contract the body portion, the first and second segments facilitating a reduction in overall compressive forces on the body portion during contracting of the body portion by the contracting member.
38. The apparatus according to embodiment 37, wherein the second segment is disposed contiguously with the first segment.
39. The apparatus according to embodiment 37, wherein the first segment has an elasticity that is different from an elasticity of the second segment.
40. The apparatus according to any one of embodiments 37 to 39, wherein the first segment is configured for implantation at an annulus of a cardiac valve of a patient between a vicinity of an anterolateral commissure and P2, and wherein the second segment is configured for implantation between P2 and a posterolateral commissure.
41. The apparatus according to embodiments 37 to 40, wherein the first segment has a greatest first diameter, and wherein the second segment has a second diameter that is smaller than the first diameter following stretching of the second segment.
42. Apparatus, comprising:
   an annuloplasty structure comprising:
   a body portion being shaped to define:
      a first segment; and
      at least one telescoping second segment being configured to be disposed at least in part within a lumen of the first segment, the at least one telescoping second segment being moveable proximally and distally along a longitudinal axis of the body portion; and
   a contracting member coupled to the body portion, the contracting member being configured to contract the body portion, the at least one telescoping second segment facilitating a reduction in overall compressive forces on the body portion during contracting of the body portion by the contracting member.
43. The apparatus according to embodiment 42, wherein the first segment is configured for implantation at an annulus of a cardiac valve of a patient between a vicinity of an anterolateral commissure and P2, and wherein the second segment is configured for implantation between P2 and a posterolateral commissure.
44. The apparatus according to any one of embodiments 42 to 43, wherein the at least one telescoping second segment comprises at least first and second telescoping segments disposed contiguously, the second telescoping segment being configured to be disposed at least in part within a lumen of the first telescoping segment, the second telescoping second segment being moveable proximally and distally along a longitudinal axis of the body portion.
45. The apparatus according to any one of embodiments 42 to 44, wherein the first segment comprises a first coupling element, and wherein the at least one telescoping second segment comprises a second coupling element, and wherein the first and second coupling elements are configured to be coupled together following movement of the at least one telescoping second segment with respect to the first segment.
46. The apparatus according to embodiment 45, wherein the first segment and the at least one telescoping second segment are shaped so as to define respective lumens, and wherein the first and second coupling elements are ring-shaped.
47. Apparatus for use with tissue of a heart of a subject, the apparatus comprising:
   an elongate annuloplasty structure, comprising a flexible body portion that comprises a tubular wall that defines a lumen along the body portion, the wall comprising:
      a sleeve; and
      a helical member, stiffer than the sleeve, and coupled to the sleeve, the helical member defining a plurality of helical turns extending in a helical path along and around the lumen;
   a plurality of anchors, each of the anchors comprising a head and a tissue-engaging element, and being dimensioned to be advanceable along the lumen; and
   at least one anchor driver, configured to, for each of the anchors:
      reversibly couple the head,
      advance the anchor along the lumen, and
      use the anchor to secure a respective helical turn to the tissue by driving the tissue-engaging element of the anchor, from the lumen, through the wall, and into the tissue, such that the helical turn becomes sandwiched between the head and the tissue.
48. The apparatus according to embodiment 47, wherein the tissue-engaging element of each of the anchors is configured to pierce the helical member, and wherein the at least one anchor driver is configured to, for each of the anchors, use the anchor to secure the respective helical turn to the tissue by driving the tissue-engaging element of the anchor from the lumen, through the helical member at the helical turn, and into the tissue, such that the helical turn becomes sandwiched between the head and the tissue.
49. The apparatus according to embodiment 47, wherein the at least one anchor driver is configured to, for each of the anchors, use the anchor to secure the respective helical turn to the tissue by driving the tissue-engaging element of the anchor from the lumen, through the wall adjacent to the helical turn, and into the tissue, such that the helical turn becomes sandwiched between the head and the tissue.
50. The apparatus according to any one of embodiments 47 to 49, wherein the at least one anchor driver is configured to intracorporeally advance each of the anchors into the lumen and along the lumen.
51. The apparatus according to any one of embodiments 47 to 50, wherein each of the anchors is dimensioned to be advanceable through the lumen while the tissue-engaging element of the anchor is disposed distally to the head of the anchor.
52. The apparatus according to any one of embodiments 47 to 51, wherein the helical member comprises at least one of a fabric, a polymer, a metal, and a shape memory material.
53. The apparatus according to any one of embodiments 47 to 52, further comprising an elongate contracting member extending along and coupled to the body portion, the contracting member being configured to contract the body portion upon being tensioned.
54. The apparatus according to embodiment 53, wherein the contracting member extends through the lumen, radially inward from the helical member.
55. The apparatus according to any one of embodiments 53 to 54, further comprising an actuatable adjustment mechanism, coupled to the body portion and to the contracting member, and configured to, upon actuation, contract the body portion by tensioning the contracting member.
56. The apparatus according to any one of embodiments 47 to 55, wherein the apparatus further comprises a tool that comprises an anchor channel that extends into the lumen, and wherein the at least one anchor driver is configured to advance the anchor along the lumen by advancing the anchor through the anchor channel.
57. The apparatus according to embodiment 56, wherein the anchor channel has a distal opening, and wherein the anchor driver is configured to advance the anchor out of a distal opening of the anchor channel and through a part of the wall that is directly in front of the distal opening.
58. The apparatus according to embodiment 56, wherein the tool is configured to transluminally deliver the annuloplasty structure to the heart while the anchor channel is disposed within the lumen.
59. The apparatus according to embodiment 56, wherein the tool is configured to progressively advance the body portion off of the anchor channel.
60. The apparatus according to embodiment 56, wherein the tool comprises a protrusion that extends radially outward from the anchor channel, and engages the helical member, inhibiting the body portion from sliding off of the channel.
61. The apparatus according to embodiment 60, wherein the tool is configured to facilitate control of advancement of the body portion off of the anchor channel via revolution of the protrusion around a central longitudinal axis of the anchor channel.
62. The apparatus according to embodiment 61, wherein the protrusion is one of rigidly fixed to the anchor channel, movable with respect to the anchor channel, and longitudinally slidable with respect to the anchor channel.
63. Apparatus, comprising:
   an annuloplasty structure comprising:
   a body portion comprising flexible material comprising a plurality of interwoven fibers, the body portion having a longitudinal axis, the body portion having at least a first longitudinal section comprising:
   a first portion of the plurality of interwoven fibers oriented at an angle of 20 - 75 degrees with respect to the longitudinal axis of the annuloplasty ring body portion; and
   a second portion of the plurality of interwoven fibers oriented at an angle of 110 - 160 degrees with respect to the longitudinal axis of the body portion.
64. The apparatus according to embodiment 63, further comprising a contracting member coupled to the body portion, the contracting member being configured to contract at least the body portion.
65. The apparatus according to any one of embodiments 63 to 64, wherein the body portion has at least a second longitudinal section adjacent the first longitudinal section, the second longitudinal section comprising:
   a third portion of the plurality of interwoven fibers oriented at an angle of 0 - 15 degrees with respect to the longitudinal axis of the body portion; and
   a fourth portion of the plurality of interwoven fibers oriented at an angle of 75 - 90 degrees with respect to the longitudinal axis of the body portion.
66. The apparatus according to embodiment 65, wherein the first longitudinal section has a compressibility that is greater than a compressibility of the second longitudinal section.
67. The apparatus according to embodiment 65, further comprising at least one tissue anchor, wherein the second longitudinal section is designated for coupling thereto the at least one tissue anchor.
68. A method performed on a simulation, comprising:
   delivering an annuloplasty structure to a simulated annulus of a simulated heart valve, the annuloplasty structure including:
      a body portion comprising flexible material, the body portion being shaped to define:
         one or more controllably-expandable sections, each one of the one or more controllably-expandable sections having a respective first length;
         one or more control wires coupled to the one or more controllably-expandable sections, the one or more control wires being movable to expand each of the one or more controllably-expandable sections to assume a respective second length that is greater than the respective first length; and
      a contracting member coupled to the body portion, the contracting member being configured to contract the body portion independently of the one or more control wires; and
   controllably expanding the one or more controllably-expandable sections by moving the one or more control wires.
69. A method, comprising:
   delivering an annuloplasty structure to a simulated annulus of a simulated heart valve, the an annuloplasty structure including a body portion comprising flexible material, the body portion being shaped to define first and second controllably-expandable sections, each one of the first and second controllably-expandable sections having a respective first length;
   controllably expanding at least the first controllably-expandable section to a second length that is greater than the first length; and
   fixing the second controllably-expandable section to maintain the first length of the second controllably-expandable section and prevent the second controllably-expandable section from expanding.
70. The method according to embodiment 69, wherein fixing the second controllably-expandable section comprises driving a tissue anchor through material of the second controllably-expandable section.
71. The method according to any one of embodiments 69 to 70, wherein each one of the first and second controllably-expandable sections comprises a respective part of the flexible material of the body portion, the part being folded.
72. A method, comprising:
   delivering an annuloplasty structure to a simulated annulus of a simulated heart valve, the annuloplasty structure including a body portion including flexible material;
   using an anchor-delivery system, anchoring a first section of the annuloplasty ring body portion to simulated tissue of the simulated annulus using a first tissue anchor;
   subsequently to the anchoring, using the anchor-delivery system, compressing a second section of the body portion toward the first section of the body portion; and
   subsequently to the compressing of the second section, using the anchor-delivery system, driving a second tissue anchor through the body portion and into simulated tissue of the annulus, and by driving the second tissue anchor, maintaining compression of the second section of the body portion.
73. The method according to embodiment 72, wherein compressing the second section of the body portion comprises reversibly engaging the anchor-delivery system with the second section.
74. The method according to any one of embodiments 72 to 73, wherein subsequently to the maintaining of the compression of the second section, contracting the body portion using a contracting member coupled to the flexible material of the body portion.
75. A method, comprising:
   delivering to a simulated annulus of a simulated heart valve an annuloplasty ring structure including an annuloplasty ring body portion having opposite first and second end portions, the first end portion having a greatest cross-sectional measurement that is greater than a greatest cross-sectional measurement of the second end portion;
   anchoring the annuloplasty ring to the simulated annulus; and
   subsequently to the anchoring, folding the second end portion toward the first end portion.
76. The method according to embodiment 75, wherein the annuloplasty ring body portion is shaped so as to define a lumen, and wherein folding the second end portion comprises pushing the second end portion into the lumen toward the first end portion.
77. The method according to any one of embodiments 75 to 76, wherein the greatest cross-sectional measurement of the first end portion is 4 to 6 mm, and the greatest cross-sectional measurement of the second end portion is 3.0 to 3.9 mm.
78. A method, comprising:
   delivering to a simulated annulus of a simulated heart valve an annuloplasty structure including:
      a body portion comprising flexible material, the body portion having a first longitudinal length during the delivering; and
      a contracting member coupled to the body portion, the contracting member being configured to contract the body portion;
   subsequently to the delivering, shortening at least a portion of the body portion to a second longitudinal length shorter than the first longitudinal length, independently of the contracting member, while maintaining an entirety of the body portion intact; and
   subsequently to the shortening, remodeling the simulated annulus by contracting the body portion using the contracting member.
79. The method according to embodiment 78, further comprising attaching the body portion to the simulated annulus.
80. The method according to any one of embodiments 78 to 79, wherein shortening the portion of the body portion comprises shortening the portion of the body portion subsequently to the attaching.
81. The method according to embodiment 80, wherein shortening the portion of the body portion subsequently to the attaching comprises allowing a deformable region of the body portion to assume a deformed shape.
82. The method according to embodiment 60, wherein shortening the portion of the body portion comprises folding a second end portion of the body portion toward a first end portion of the body portion.

## Claims

1. Apparatus, comprising:
an annuloplasty structure having a longitudinal axis, the annuloplasty structure comprising:
a primary body portion;
one or more linking segments removably coupled to the primary body portion;
a first coupling coupled to a first end of the primary body portion;
a second coupling coupled to a first end of a first linking segment of the one or more linking segments that is disposed adjacent to the first coupling of the first end of the primary body portion, the first and second couplings being shaped so as to mate with each other; and
a decoupling-prevention element extending longitudinally along at least the first and second couplings in order to prevent decoupling of the first and second couplings,
wherein removal of the decoupling-prevention element from at least the first and second couplings facilitates decoupling of the first and second couplings.

2. The apparatus according to claim 1, wherein at least one of the primary body portion and the one or more linking segments is flexible.

3. The apparatus according to any one of claims 1 to 2, further comprising an elongate contracting member extending along a longitudinal length of the primary body portion and along the one or more linking segments.

4. The apparatus according to any one of claims 1 to 2, wherein the decoupling-prevention element comprises an elongate contracting member extending along a longitudinal length of the primary body portion and the one or more linking segments.

5. The apparatus according to any one of claims 1 to 4, further comprising a contracting mechanism, coupled to the annuloplasty structure and configured to contract at least a portion of the implant structure.

6. The apparatus according to any one of claims 1 to 5, wherein:
the first coupling is shaped so as to define a first lumen and comprises one or more radially-moveable projections, the one or more radially-moveable projections being radially moveable in response to a force applied thereto, and
the second coupling is shaped so as to define a second lumen and a negative space shaped so as to receive the one or more radially-moveable projections.

7. The apparatus according to any one of claims 1 to 5, wherein:
the first coupling is shaped so as to define a first lumen and comprises one or more radially-moveable projections, the one or more radially-moveable projections being biased to collapse radially inwardly in an absence of force applied thereto;
the second coupling is shaped so as to define a second lumen and a negative space shaped so as to receive the one or more radially-moveable projections; and
the decoupling-prevention element comprises an elongate tube configured to maintain the one or more radially-moveable projections within the negative space of the second coupling in order to prevent decoupling of the first and second couplings while a portion of the elongate tube passes through the first and second lumens of the respective first and second couplings.

8. The apparatus according to claim 7, wherein the elongate tube comprises a portion of an anchor-delivery system, and wherein the anchor-delivery system is configured to deploy a tissue anchor through the one or more linking segments.

9. The apparatus according to claim 7, wherein:
the one or more linking segments comprises at least the first linking segment and at least a second linking segment,
the first linking segment comprises a third coupling coupled to a second of the first linking segment,
the second linking segment comprises a fourth coupling coupled to a first end of the second linking segment that is disposed adjacent to the first linking segment, the fourth coupling being shaped so as to mate with the third coupling,
the decoupling-prevention element extends longitudinally at least through the third and fourth couplings in order to prevent decoupling of the third and fourth couplings, and
removal of the decoupling-prevention element from at least the third and fourth couplings facilitates decoupling of the third and fourth couplings.
